# EUROPEAN PATENT APPLICATION

(11) **EP 4 393 954 A1**
(43) Date of publication of application: **03.07.2024**
(21) Application number: 22871664.3
(22) Date of filing: 11.08.2022
(51) Int. Cl.: C07K 16/28, C12N 15/13, A61K 39/395, A61P 35/00

(54) **ANTI-TNFR2 MONOCLONAL ANTIBODY AND APPLICATION THEREOF**

(30) Priority: 22.09.2021 CN 202111105848
(71) Applicant: Shanghai Celgen Bio-Pharmaceutical Co., Ltd., Shanghai 201203 (CN)
(72) Inventor: CAI, Zeling, Shanghai 201203 (CN); CHEN, Yi, Shanghai 201203 (CN); WANG, Yaling, Shanghai 201203 (CN); HE, Qiaoqiao, Shanghai 201203 (CN)
(74) Representative: Santoro, Sofia
(86) International application number: PCT/CN2022/111634
(87) International publication number: WO 2023/045606

(57) **Abstract**

Provided are an anti-TNFR2 monoclonal antibody and an application thereof, Specifically, provided is an TNFR2-targeted monoclonal antibody or an antigen binding fragment thereof, which specifically binds to a cysteine-rich domain (CRD) of human TNFR2. Furthermore, provided are a murine antibody, human-mouse chimeric antibody, and humanized antibody of the TNFR2 antibody, a pharmaceutical composition containing the antibody, and a use thereof in a related disease diagnostic agent and therapeutic drug. The anti-TNFR2 monoclonal antibody has high specificity and high affinity against human TNFR2, can further achieve an immunomodulatory effect, and is used in applications such as anti-tumor, anti-infection and/or anti-autoimmune diseases.

## Description

### Technical Field

The present disclosure belongs to the field of biotechnology and medicine. Specifically, the present disclosure relates to the field of monoclonal antibody, especially an anti-TNFR2 monoclonal antibody and application thereof.

### Background

TNFRSF1B (TNF receptor superfamily member 1b or CD120b) is a member of the TNF receptor superfamily, also known as TNFR2. Unlike TNFR1, another member of the TNF receptor superfamily, which is widely distributed on the surface of most cells *in vivo,* TNFR2 is mainly expressed in immune cells, also in some tumor cells.

TNFR2 is a type I transmembrane glycoprotein, consisting of 439 amino acids (accession number is UniProtKB-P01857), wherein the first 235 amino acids constitute the extracellular region to form four cysteine-rich domains (CRD). Each CRD comprises 6 cysteines, forming 3 pairs of disulfide bonds. The unique spatial structure of CRD determines the specific binding between TNF and the receptor. CRD2 and CRD3 are the portions where the receptor binds to TNF (Mukai et al. Science Signaling 148:1-10, 2010). Although CRD1 and CRD4 are not directly involved in the recognition and binding of TNF, their absence results in a significant decrease in the receptor's binding ability to TNF.

TNF-α, a ligand of TNFRSF1B, is mainly involved in apoptosis by recognizing TNFR1, and relies on TNFR2 to perform functions related to immune system regulation. Studies have found that the loss of TNFR2 led to reduced function of regulatory T cells (Treg) in mice, which aggravated autoimmune diseases and colitis. In human, reduced expression or reduced binding of TNFR2 to TNF-α interferes with TNFR2-mediated regulatory T cell signaling, and is strongly associated with autoimmune diseases such as systemic lupus erythematosus (SLE), Crohn disease, and ulcerative colitis. In addition, TNFR2 is highly upregulated in cancer, and in tumor microenvironment, TNFR2 is expressed on certain tumor cells and immune cells, including Treg cells, CD8+ killer T cells, Breg cells, NK cells, myeloid-derived suppressor cells (MDSCs), and other types of cells, such as endothelial cells and their precursors endothelial progenitor cells (ECs, EPCs) and mesenchymal stem cells (MSCs). These cells play an important role in tumor immune escape, tumor growth, and tumor progression.

However, how the TNFR2 signaling pathway regulates the biological functions and key signaling events of these cells in tumor microenvironment is unclear, and further research is required (Yang Yang, et al., TNFR2: Role in Cancer Immunology and Immunotherapy, Immunotargets Ther. 2021; 10: 103-122).

For now, TNFR2 has been found in more than 25 types of tumors, comprising, for example: lung cancer, adrenal cortical cancer, bladder urothelial cancer, cervical cancer, cholangiocarcinoma, colon cancer, esophageal cancer, glioma, head and neck squamous cell carcinoma, renal clear cell carcinoma, renal papillary cell carcinoma, acute myeloid leukemia, low grade glioma of brain, hepatocellular carcinoma, ovarian cancer, pancreatic adenocarcinoma, chromocytoma and paraganglioma, prostate cancer, rectal adenocarcinoma, sarcoma, melanoma, gastric adenocarcinoma, testicular cancer, thyroid cancer, uterine cancer, etc. (Vanamee ES, Faustman DL, TNFR2: A Novel Target for Cancer Immunotherapy. [Trends Mol Med. 2017] Trends Mol Med. 2017 Nov; 23(11):1037-1046.

TNFR2 is considered to be a promising target for cancer immunotherapy, with its high expression in Tregs located in tumor and on the surface of various human tumor cells (Williams GS et al., Oncotarget. 2016; 7(42): 68278-68291; Vanamee ES et al., Trends in Molecular Medicine, 2017, vol. 23, issue 11, 1037-1046).

At present, two major classes of anti-TNFR2 antibodies with therapeutic potential are TNFR2 antagonists and TNFR2 agonists, and they have different properties and mechanisms of action. Preclinical data have shown that TNFR2 antagonists can be used to treat proliferative diseases such as cancer or infectious diseases. WO2014/124134 disclosed a TNFR2 antagonist that selectively binds to an epitope in the sequence KQEGCRLCAPLRKCRPGGV, having fuctions of enriching lymphoimmune cells and specifically killing Treg cells in tumor microenvironment. WO2017/197331 disclosed an antagonistic TNFR2 antibody with a specific sequence of complementary determining region-heavy Chain 3(CDR-H3), which could reduce or inhibit Treg proliferation and/or promote the proliferation of T effector cells. On the other aspect, agonistic TNFR2 antibody can activate Treg cells and then inhibit effector T cells, thus contributing to the treatment of immune diseases. For example, WO2017/040312 disclosed an agonistic anti-TNFR2 antibody, which could promote TNFR2 signal transduction and promote the expansion or proliferation of Treg, so as to treat autoimmune diseases, thereby providing a new target for immunotherapy. In addition, it has been shown that agonistic TNFR2 antibodies can bind to any TNFR2-expressing immune cell, such as CD8+ T cells. CN112996812A disclosed an agonistic TNFR2 antibody that could enhance the production of IFN-γ in NK cells, and promote the activation of CD4+ T cells. In mouse tumor models, this agonistic TNFR2 antibody could also increase the infiltration of CD8+ T cells and reduce the number of Treg in tumors, showing potent anti-tumor effects.

In summary, TNFR2 is a promising new immunotherapy target for the treatment of a variety of tumors and immune-related diseases. The research and development of antibody drugs that can efficiently target the TNFR2 signaling pathway with clinical efficacy has great application potential and commercial value.

### Summary of the Invention

The present invention provides an antibody and antigen binding fragment thereof that bind to TNFR2, as well as related products and applications thereof.

In some aspects of the present invention, a monoclonal antibody or antigen binding fragment thereof targeting TNFR2 is provided, which specifically binds to the cysteine-rich domain of human TNFR2.

In some aspects of the present invention, a nucleic acid molecule encoding the monoclonal antibody or antigen-binding fragment thereof of the present application, or a vector or a host cell containing the nucleic acid molecule is provided.

In some aspects of the present invention, an immunoconjugate is provided, which comprises:
(a) the monoclonal antibody or antigen binding fragment thereof of the present application;
(b) a coupling moiety selected from the group consisting of: a drug, a toxin, a cytokine, a radioactive isotope or an enzyme; and
(c) optionally, a linker.

In some aspects of the present invention, a chimeric antigen receptor is provided, which comprises an extracellular domain and an intracellular domain, wherein the extracellular domain comprises the monoclonal antibody or antigen binding fragment thereof of the present application.

In some aspects of the present invention, a nucleic acid molecule and a construct or a vector containing the nucleotide molecule is provided, wherein the nucleic acid molecule contains a sequence encoding the chimeric antigen receptor of the present application.

In some aspects of the present invention, a transformed immune cell is provided, which expresses the chimeric antigen receptor of the present application, or which is transformed by the nucleic acid molecule, construct, or vector of the present application, for example, the immune cell is selected from T cell, such as αβ T cell, γδ T cell, or NK T cell, or T cell derived from pluripotent cell.

In some aspects of the present invention, a composition is provided, wich comprises: the monoclonal antibody or antigen binding fragment thereof, nucleic acid molecule, vector or host cell or immunoconjugate, chimeric antigen receptor, encoding nucleic acid molecule thereof, construct or vector, and/or transformed immune cell of the present application; and optionally, a physiologically or pharmaceutically acceptable carrier or excipient.

In some aspects of the present invention, use of the monoclonal antibody or antigen binding fragment thereof, nucleic acid molecule, vector or host cell or immunoconjugate, chimeric antigen receptor, encoding nucleic acid molecule thereof, construct or vector, transformed immune cell and/or composition of the present application in the preparation of a drug for positively regulating activity of immune cells and/or enhancing immune response, such as in the preparation of a drug for preventing and/or treating tumors, infections or infectious diseases is provided.

In some aspects of the invention, a method for positively regulating the activity of immune cells and/or enhancing immune response (such as a method for preventing and/or treating tumors, infections or infectious diseases) is provided, which comprises a step of administering the monoclonal antibody or antigen binding fragment thereof, nucleic acid molecule, vector or host cell or immunoconjugate, chimeric antigen receptor, encoding nucleic acid molecule thereof, construct or vector, transformed immune cell and/or composition of the present application to a subject in need thereof.

In some aspects of the present invention, use of the monoclonal antibody or antigen binding fragment thereof, nucleic acid molecule, vector or host cell or immunoconjugate, chimeric antigen receptor, encoding nucleic acid molecule thereof, construct or vector, transformed immune cell and/or composition of the present application in the preparation of a drug for negatively regulating activity of immune cells and/or reducing immune response, such as in the preparation of a drug for prevention and/or treatment of autoimmune diseases, cytokine storms, or allergic diseases is provided.

In some aspects of the present invention, a method for negatively regulating activity of immune cells and/or reducing immune response (such as a method for preventing and/or treating autoimmune diseases, cytokine storms, or allergic diseases) is provided, which comprises a step of administering the monoclonal antibody or antigen-binding fragment thereof, nucleic acid molecule, vector or host cell or immunoconjugate, chimeric antigen receptor, encoding nucleic acid molecule thereof, construct or vector, transformed immune cell, and/or composition of the present application to a subject in need thereof.

In some aspects of the present invention, the monoclonal antibody or antigen binding fragment thereof, nucleic acid molecule, vector or host cell or immunoconjugate, chimeric antigen receptor, encoding nucleic acid molecule thereof, construct or vector, transformed immune cell and/or composition of the present application are provided.

Those skilled in this field can combine the aforementioned technical solutions and features freely without departing from the inventive concept and protection scope of the present invention. Other aspects of the present invention are obvious to those skilled in the art due to the disclsoure herein.

### Brief Description of the Drawings

The following is a further explanation of the present invention with the drawings, wherein these drawings are only intended to illustrate the embodiments of the present invention and not to limit its scope.
**Figure 1****:** ELISA research of 8 strains of anti-human TNFR2 mouse monoclonal antibodies (clone numbers 16D2, 27F6, 29A1, 7F2, 11G2, 12E12, 13F5, and 18A2) binding to recombinant human TNFR2 (hTNFR2) *in vitro.*
**Figure 2****:** ELISA research of 8 strains of anti-human TNFR2 mouse monoclonal antibodies (clone numbers 16D2, 27F6, 29A1, 7F2, 11G2, 12E12, 13F5, and 18A2) binding to recombinant mouse TNFR2 (mTNFR2) *in vitro.*
**Figure 3****:** ELISA research of 8 strains of anti-human TNFR2 mouse monoclonal antibodies (clone numbers 16D2, 27F6, 29A1, 7F2, 11G2, 12E12, 13F5, and 18A2) binding to recombinant cynomolgus monkey TNFR2 (Cyno. TNFR2) *in vitro.*
**Figure 4****:** Flow cytometry analysis of 6 strains of anti-human TNFR2 mouse monoclonal antibodies (clone numbers 16D2, 27F6, 29A1, 7F2, 11G2, and 12E12) binding to CHO cell membrane overexpressing TNFR2.
**Figure 5****:** ELISA research of 5 strains of anti-human TNFR2 mouse monoclonal antibodies (clone numbers 27F6, 11G2, 12E12, 27F6, and 33C12) binding to mutated human TNFR2 antigen *in vitro.*
**Figure 6****:** ELISA research of 6 strains of anti-human TNFR2 human-mouse chimeric monoclonal antibodies (numbers 27F6-hFc, 29A1 hFc, 7F2 hFc, 11G2 hFc, 12E12-hFc, and 33C12-hFc) binding to recombinant human TNFR2 *in vitro.*
**Figure 7****:** Flow cytometry analysis of 6 strains of human-mouse chimeric TNFR2 monoclonal antibodies (27F6-hFc, 29A1 hF, 7F2 hFc, 11G2 hFc, 12E12-hFc, and 33C12-hFc) binding to TNFR2 positive Jurkat cell membrane.
**Figure 8****:** ELISA research of 6 strains of human-mouse chimeric TNFR2 monoclonal antibodies (27F6-hFc, 29A1 hF, 7F2 hFc, 11G2 hFc, 12E12-hFc, and 33C12-hFc) and TNFα fusion proteins on the competitive binding to recombinant human TNFR2.
**Figure 9****:** FACS research of 6 strains of human-mouse chimeric TNFR2 monoclonal antibodies (27F6-hFc, 29A1 hF, 7F2 hFc, 11G2 hFc, 12E12-hFc, and 33C12-hFc) and TNFα fusion proteins on the competitive binding to recombinant human TNFR2 on cell membrane.
**Figure 10****:** Flow cytometry analysis of human-mouse chimeric TNFR2 monoclonal antibodies binding to human TNFR2 on the surface of T cell.
**Figure 11****:** The human-mouse chimeric TNFR2 monoclonal antibodies promoted Jurkat-TNFR2 to secret IL2.
**Figure 12****:** The effect of human-mouse chimeric TNFR2 monoclonal antibodies on the activity of Jurkat TNFR2 cells.
**Figure 13****:** Flow cytometry analysis of TNFR2 expression in Jeko-1 cells.
**Figure 14****:** The effect of human-mouse chimeric TNFR2 monoclonal antibodies on the activity of Jeko-1 cells.
**Figure 15****:** The effect of TNFα and human-mouse chimeric TNFR2 monoclonal antibodies on the activity of Jeko-1 cells.
**Figure 16****:** The binding of TNFR2 monoclonal antibodies to T cells of hTNFR2 konck-in mice.
**Figure 17****:** The line graph of tumor volume over time in mice treated with TNFR2 mouse monoclonal antibodies.
**Figure 18****:** SDS-PAGE detection of non-reducing and reducing humanized antibodies.
**Figure 19****:** ELISA research of *in vitro* binding activity of humanized anti-human TNFR2 monoclonal antibodies to recombinant human TNFR2:
   Figure 19A: *In vitro* binding of anti-human TNFR2 humanized monoclonal antibody 11G2 to recombinant human TNFR2;
   Figure 19B: *In vitro* binding of anti-human TNFR2 humanized monoclonal antibody 33C12 to recombinant human TNFR2;
   Figure C: *In vitro* binding of anti-human TNFR2 humanized monoclonal antibody 12E122 to recombinant human TNFR2 (the first test);
   Figure D: *In vitro* binding of anti-human TNFR2 humanized monoclonal antibody 12E12 to recombinant human TNFR2 (the second test).
**Figure 20****:** Flow cytometry analysis on the binding of humanized antibodies to TNFR2 protein on cell membrane:
   Figure 20A: *In vitro* binding of anti-human TNFR2 humanized monoclonal antibody 11G2 to cell membrane TNFR2;
   Figure 20B: *In vitro* binding of anti-human TNFR2 humanized monoclonal antibody 33C12 to cell membrane TNFR2;
   Figure 20C: *In vitro* binding of anti-human TNFR2 humanized monoclonal antibody 12E22 to cell membrane TNFR2.

### Detailed Description

The present invention discloses antibodies that specifically bind to TNFR2 with high affinity and have therapeutic effects on various tumors and immune system related diseases.

The antibody described in the present invention can have antagonistic or agonistic effect on signals mediated by TNFR2, some of which can block the interaction of TNFα and TNFR2. The antibodies of the present invention can regulate the activity of cells expressing TNFR2, and some antibodies can significantly promote TNFα-induced apoptosis of TNFR2 expressing cells, and some antibodies have a significant protective effect on TNF-induced cell apoptosis. The antibody of the present invention can also enhance the cytokine release of immune cells, improve immune cell activity, and enhance immune response. Through the anti-colon cancer MC-38 test in mice, it has been proven that the antibody of the present invention has a significant inhibitory effect on tumors and has great potential for treating tumors.

All numerical ranges provided herein are aimed to clearly include all values that fall between the endpoints of the range and the numerical ranges between them. The features mentioned in the present invention or in the embodiments can be combined. All features disclosed in this specification can be combined with any combination form, and each feature disclosed in the specification can be replaced by any alternative feature that provides the same, equal, or similar purpose. Therefore, unless otherwise specified, the disclosed features are only general examples of equal or similar features.

As used herein, "comprising", "having" or "including" comprises "comprising", "mainly consisting of", "basically consisting of", and "consisting of"; and "mainly consisting of", "basically consisting of", and "consisting of" belong to the subordinate concepts of" comprising", "having", or "including".

### Monoclonal Antibody and Preparation thereof

The term "monoclonal antibody (mAb)" used herein refers to antibodies obtained from a basically homogeneous population, where the individual antibodies within the population are identical, except for a few possible natural mutations. A monoclonal antibody targets a single antigen site with high specificity. Moreover, unlike conventional polyclonal antibody formulations (usually with different antibodies targeting different determinant clusters), each monoclonal antibody targets a single determinant cluster on the antigen. In addition to their specificity, the advantage of monoclonal antibodies is that they are synthesized through hybridoma culturing and will not be contaminated by other immunoglobulins. The modifier "monoclonal" indicates the characteristics of the antibody, which is obtained from a basically homogeneous population of antibodies, and should not be interpreted as requiring any special method to produce the antibody.

As used herein, the term "antibody" or "immunoglobulin" refers to an isotetran glycoprotein of approximately 150,000 Daltons with the same structural characteristics, consisting of two identical light chains (L) and heavy chains (H). Each light chain is connected to the heavy chain through a covalent disulfide bond, and the number of disulfide bonds between heavy chains varies from different immunoglobulin isotypes. Each heavy and light chain also has regularly spaced intra-chain disulfide bonds. Each heavy chain has a variable region (VH) at one end, followed by multiple constant regions. Each light chain has a variable region (VL) at one end and a constant region at the other end. The constant region of the light chain is located opposite to the first constant region of the heavy chain, and the variable region of the light chain is located opposite to the variable region of the heavy chain. Special amino acid residues form an interface between the variable regions of the light and heavy chains.

As used herein, the term "variable" refers to certain parts of the variable region in antibody that differ in sequence, forming the binding specificity of various specific antibodies to their specific antigens. However, variability is not evenly distributed throughout the variable region of the antibody. It is concentrated in three fragments in the variable regions of light and heavy chains, known as complementary determining regions (CDRs) or hypervariable regions. The more conservative part of the variable region is called the framework region (FR). The variable regions of natural heavy chains and light chains each comprises four FR regions, which roughly exhibit a β-folding configuration, connected by three CDRs forming a connecting ring, and can form a partially b-folding structure in some cases. The CDRs in each chain are tightly packed together through the FR regions and form the antigen binding site of antibody with the CDRs in another chain (see Kabat et al., NIH Public. No. 91-3242, Volume I, pages 647-669 (1991)). The constant regions do not directly participate in the binding of antibodies and antigens, but they show different effector functions, such as antibody dependent cytotoxicity.

The present disclosure provides an antibody that specifically binds to human TNFR2, comprising a mouse antibody, a chimeric antibody, and a humanized antibody. The monoclonal antibody disclosed herein can also be a chimeric antibody or a humanized antibody. In the present disclosure, unless otherwise specified, "m" represents the mouse-derived portion and "h" represents the human-derived portion. In some embodiments, the antibody comprises a heavy chain variable region (VH), wherein the heavy chain variable region comprises three complementary determining regions (CDRs), and the antibody comprises a light chain variable region (VL), and the light chain variable region comprises three CDRs.

As used herein, the term "sequence identity" or "identity%" refers to the percentage of identical residues (such as amino acids or nucleic acids) between the candidate sequence and the reference sequence after aligning the sequence and (if necessary) introducing spaces to obtain the maximum sequence identity percentage. For example, As used herein, "at least 70% sequence identity" refers to the sequence identity between the candidate sequence and the reference sequence reaching more than 70%, such as 70, 75, 80, 85, 90, 91, 92, 93, 94, 95, 96, 97, 98, 99, 100%, or any numerical point or range of identity.

In some embodiments, the VH amino acid sequence of the antibody is selected from: SEQ ID NO: 1, 9, 17, 25, 33, 41, 49, 57, 65, or humanized sequences thereof, such as SEQ ID NO: 73, 74, 75, 76, 77, 81, 82, 83, 92, 93, 94, 96, 97, 99, 100, or 101.

In some embodiments, the VL amino acid sequence of the antibody is selected from: SEQ ID NO: 5. 13, 21, 29, 37, 45, 53, 61, 69, or humanized sequences thereof, such as SEQ ID NO: 78, 79, 80, 84, 85, 86, 102, 103, 104, 105, or 106.

In some embodiments, the antibody comprises any combination of the VH amino acid sequences and the VL amino acid sequences selected from above. In some embodiments, the combination of VH and VL amino acid sequences of the antibody is: SEQ ID NOs: 1 and 5; SEQ ID NOs: 9 and 13; SEQ ID NOs: 17 and 21; SEQ ID NOs: 25 and 29; SEQ ID NOs: 33 and 37; SEQ ID NOs: 41 and 45; SEQ ID NOs: 49 and 53; SEQ ID NOs: 57 and 61; SEQ ID NOs: 65 and 69; or humanized sequences thereof, such as the combination of any one of SEQ ID NOs: 73-77 with SEQ ID NO: 78, 79, or 80; the combination of any one of SEQ ID NOs: 81-83 with SEQ ID NO: 84, 85, or 86; the combination of any one of SEQ ID NOs: 92-94, 96-97, and 99-101 with any one of SEQ ID NOs: 102-106.

In some embodiments, the amino acid sequences of VH CDR1, 2, and 3 of the antibody are selected from the group consisting of: SEQ ID NOs: 2, 3, and 4, respectively; SEQ ID NOs: 10, 11 and 12, respectively; SEQ ID NOs: 18. 19 and 20, respectively; SEQ ID NOs: 26, 27, and 28, respectively; SEQ ID NOs: 34, 35, and 36, respectively; SEQ ID NOs: 42, 43, and 44, respectively; SEQ ID NOs: 50, 51, and 52, respectively; SEQ ID NOs: 58, 59, and 60, respectively; SEQ ID NOs: 66, 67, and 68, respectively.

In some embodiments, the amino acid sequences of VH CDR1, 2, and 3 of the antibody are SEQ ID NOs: 2, 3, and 4, and the VH sequence has at least 70% (such as 75, 80, 85, 90, 91, 92, 93, 94, 95, 96, 97, 98, 99, 100% or any numerical point in the range) sequence identity with SEQ ID NO: 1.

In some embodiments, the amino acid sequences of VH CDR1, 2, and 3 of the antibody are SEQ ID NOs: 10, 11, and 12, and the VH sequence has at least 70% sequence identity with SEQ ID NO: 9.

In some embodiments, the amino acid sequences of VH CDR1, 2, and 3 of the antibody are SEQ ID NOs: 18, 19, and 20, and the VH sequence has at least 70% sequence identity with SEQ ID NO: 17.

In some embodiments, the amino acid sequences of VH CDR1, 2, and 3 of the antibody are SEQ ID NOs: 26, 27, and 28, and the VH sequence has at least 70% sequence identity with SEQ ID NO: 25.

In some embodiments, the amino acid sequences of VH CDR1, 2, and 3 of the antibody are SEQ ID NOs: 34, 35, and 36, and the VH sequence has at least 70% sequence identity with SEQ ID NO: 33.

In some embodiments, the amino acid sequences of VH CDR1, 2, and 3 of the antibody are SEQ ID NOs: 34, 87, and 36, and the VH sequence (e.g., SEQ ID NO: 75) has at least 70% sequence identity with SEQ ID NO: 33.

In some embodiments, the amino acid sequences of VH CDR1, 2, and 3 of the antibody are SEQ ID NOs: 34, 88, and 36, and the VH sequence (e.g., SEQ ID NO: 76 or 77) has at least 70% sequence identity with SEQ ID NO: 33.

In some embodiments, the amino acid sequences of VH CDR1, 2, and 3 of the antibody are SEQ ID NOs: 42, 43, and 44, the VH sequence has at least 70% sequence identity with SEQ ID NO: 41.

In some embodiments, the amino acid sequences of VH CDR1, 2, and 3 of the antibody are SEQ ID NOs: 42, 95, and 44, and the VH sequence (e.g., SEQ ID NO: 94 or 100) has at least 70% sequence identity with SEQ ID NO: 41.

In some embodiments, the amino acid sequences of VH CDR1, 2, and 3 of the antibody are SEQ ID NOs: 42, 98, and 44, and the VH sequence (e.g., SEQ ID NO: 97) has at least 70% sequence identity with SEQ ID NO: 41.

In some embodiments, the amino acid sequences of VH CDR1, 2, and 3 of the antibody are SEQ ID NOs: 50, 51, and 52, and the VH sequence has at least 70% sequence identity with SEQ ID NO: 49.

In some embodiments, the amino acid sequences of VH CDR1, 2, and 3 of the antibody are SEQ ID NOs: 58, 59, and 60, and the VH sequence has at least 70% sequence identity with SEQ ID NO: 57.

In some embodiments, the amino acid sequences of VH CDR1, 2, and 3 of the antibody are SEQ ID NOs: 66, 67, and 68, and the VH sequence has at least 70% sequence identity with SEQ ID NO: 65.

In some embodiments, the amino acid sequences of VH CDR1, 2, and 3 of the antibody are SEQ ID NOs: 66, 90, and 68, and the VH sequence (e.g., SEQ ID NO: 83) has at least 70% sequence identity with SEQ ID NO: 65.

In some embodiments, the amino acid sequences of VH CDR1, 2, and 3 of the antibody are selected from the group consisting of: SEQ ID NOs: 2, 3, and 4, respectively; SEQ ID NOs: 10. 11 and 12, respectively; SEQ ID NOs: 18. 19 and 20, respectively; SEQ ID NOs: 26, 27, and 28, respectively; SEQ ID NOs: 34, 35, and 36, respectively; SEQ ID NOs: 42, 43, and 44, respectively; SEQ ID NOs: 50, 51, and 52, respectively; SEQ ID NOs: 58, 59, and 60, respectively; and SEQ ID NOs: 66, 67, and 68, respectively.

In some embodiments, the amino acid sequences of VL CDR1, 2, and 3 of the antibody are selected from the group consisting of: SEQ ID NOs: 6, 7, and 8, respectively; SEQ ID NOs: 14, 15 and 16, respectively; SEQ ID NOs: 22, 23, and 24, respectively; SEQ ID NOs: 30, 31, and 32, respectively; SEQ ID NOs: 38, 39, and 40, respectively; SEQ ID NOs: 46, 47, and 48, respectively; SEQ ID NOs: 54, 55, and 56, respectively; SEQ ID NOs: 62, 63, and 64, respectively; and SEQ ID NOs: 70, 71, and 72, respectively.

In some embodiments, the amino acid sequences of VL CDR1, 2, and 3 of the antibody are SEQ ID NOs: 6, 7, and 8, and the VL sequence has at least 70% (such as 75, 80, 85, 90, 91, 92, 93, 94, 95, 96, 97, 98, 99, 100%, or any numerical point in the range) sequence identity with SEQ ID NO: 5.

In some embodiments, the amino acid sequences of VL CDR1, 2, and 3 of the antibody are SEQ ID NOs: 14, 15, and 16, and the VL sequence has at least 70% sequence identity with SEQ ID NO: 13.

In some embodiments, the amino acid sequences of VL CDR1, 2, and 3 of the antibody are SEQ ID NOs: 22, 23, and 24, and the VL sequence has at least 70% sequence identity with SEQ ID NO: 21.

In some embodiments, the amino acid sequences of VL CDR1, 2, and 3 of the antibody are SEQ ID NOs: 30, 31, and 32, and the VL sequence has at least 70% sequence identity with SEQ ID NO: 29.

In some embodiments, the amino acid sequences of VL CDR1, 2, and 3 of the antibody are SEQ ID NOs: 38, 39, and 40, and the VL sequence has at least 70% sequence identity with SEQ ID NO: 37.

In some embodiments, the amino acid sequences of VL CDR1, 2, and 3 of the antibody are SEQ ID NOs: 38, 89, and 40, and the VL sequence (e.g., SEQ ID NO: 80) has at least 70% sequence identity with SEQ ID NO: 37.

In some embodiments, the amino acid sequences of VL CDR1, 2, and 3 of the antibody are SEQ ID NOs: 46, 47, and 48, and the VL sequence has at least 70% sequence identity with SEQ ID NO: 45.

In some embodiments, the amino acid sequences of VL CDR1, 2, and 3 of the antibody are SEQ ID NOs: 46, 107, and 48, and the VL sequence (e.g., SEQ ID NO: 106) has at least 70% sequence identity with SEQ ID NO: 45.

In some embodiments, the amino acid sequences of VL CDR1, 2, and 3 of the antibody are SEQ ID NOs: 54, 55, and 56, and the VL sequence has at least 70% sequence identity with SEQ ID NO: 53.

In some embodiments, the amino acid sequences of VL CDR1, 2, and 3 of the antibody are SEQ ID NOs: 62, 63, and 64, and the VL sequence has at least 70% sequence identity with SEQ ID NO: 61.

In some embodiments, the amino acid sequences of VL CDR1, 2, and 3 of the antibody are SEQ ID NOs: 70, 71, and 72, and the VL sequence has at least 70% sequence identity with SEQ ID NO: 69.

In some embodiments, the amino acid sequences of VL CDR1, 2, and 3 of the antibody are SEQ ID NOs: 70, 91, and 72, and the VL sequence (e.g., SEQ ID NO: 86) has at least 70% sequence identity with SEQ ID NO: 69.

In some embodiments, the amino acid sequences of VH CDR1, 2 and 3 and VL CDR1, 2 and 3 of the antibody are selected from the group consisting of: SEQ ID NOs: 2, 3 and 4, and SEQ ID NOs: 6, 7 and 8, respectively; SEQ ID NOs: 10, 11 and 12, and SEQ ID NOs: 14, 15 and 16, respectively; SEQ ID NOs: 18, 19 and 20, and SEQ ID NOs: 22, 23, and 24, respectively; SEQ ID NOs: 26, 27, and 28, and SEQ ID NOs: 30, 31, and 32, respectively; SEQ ID NOs: 34, 35, and 36, and SEQ ID NOs: 38, 39, and 40, respectively; SEQ ID NOs: 42, 43, and 44, and SEQ ID NOs: 46, 47, and 48, respectively; SEQ ID NOs: 50, 51, and 52, and SEQ ID NOs: 54, 55, and 56, respectively; SEQ ID NOs: 58, 59, and 60, and SEQ ID NOs: 62, 63, and 64, respectively; SEQ ID NOs: 66, 67, and 68, and SEQ ID NOs: 70, 71, and 72, respectively.

In some embodiments, the amino acid sequences of VH CDR1, 2 and 3, and VL CDR1, 2 and 3 of the antibody are: SEQ ID NOs: 2, 3 and 4, and SEQ ID NOs: 6, 7 and 8, and the VH sequence has at least 70% (such as 75, 80, 85, 90, 91, 92, 93, 94, 95, 96, 97, 98, 99, 100%, or any numerical point in the range) sequence identity with SEQ ID NO: 1, and the VL sequenc has at least 70% (such as 75, 80, 85, 90, 91, 92, 93, 94, 95, 96, 97, 98, 99, 100% or any numerical point in the range) sequence identity with SEQ ID NO: 5.

In some embodiments, the amino acid sequences of VH CDR1, 2 and 3 and VL CDR1, 2 and 3 of the antibody are: SEQ ID NOs: 10, 11 and 12, and SEQ ID NOs: 14, 15 and 16, and the VH sequence has at least 70% sequence identity with SEQ ID NO:9, and the VL sequence has at least 70% sequence identity with SEQ ID NO: 13.

In some embodiments, the amino acid sequences of VH CDR1, 2 and 3 and VL CDR1, 2 and 3 of the antibody are: SEQ ID NOs: 18. 19 and 20, and SEQ ID NOs: 22, 23, and 24, and the VH sequence has at least 70% sequence identity with SEQ ID NO: 17, and the VL sequence has at least 70% sequence identity with SEQ ID NO: 21.

In some embodiments, the amino acid sequences of VH CDR1, 2 and 3 and VL CDR1, 2 and 3 of the antibody are: SEQ ID NOs: 26, 27, and 28, and SEQ ID NOs: 30, 31, and 32, and the VH sequence has at least 70% sequence identity with SEQ ID NO: 25, and the VL sequence has at least 70% sequence identity with SEQ ID NO: 29.

In some embodiments, the amino acid sequences of VH CDR1, 2 and 3 and VL CDR1, 2 and 3 of the antibody are: SEQ ID NOs: 34, 35, and 36, and SEQ ID NOs: 38, 39, and 40, and the VH sequence has at least 70% sequence identity with SEQ ID NO: 33, and the VL sequence has at least 70% sequence identity with SEQ ID NO: 37.

In some embodiments, the amino acid sequences of VH CDR1, 2 and 3 and VL CDR1, 2 and 3 of the antibody are: SEQ ID NOs: 42, 43, and 44, and SEQ ID NOs: 46, 47, and 48, and the VH sequence has at least 70% sequence identity with SEQ ID NO: 41, and the VL sequence has at least 70% sequence identity with SEQ ID NO: 45.

In some embodiments, the amino acid sequences of VH CDR1, 2 and 3 and VL CDR1, 2 and 3 of the antibody are: SEQ ID NOs: 50, 51, and 52, and SEQ ID NOs: 54, 55, and 56, and the VH sequence has at least 70% sequence identity with SEQ ID NO: 49, and the VL sequence has at least 70% sequence identity with SEQ ID NO: 53.

In some embodiments, the amino acid sequences of VH CDR1, 2 and 3 and VL CDR1, 2 and 3 of the antibody are: SEQ ID NOs: 58, 59, and 60, and SEQ ID NOs: 62, 63, and 64, and the VH sequence has at least 70% sequence identity with SEQ ID NO: 57, and the VL sequence has at least 70% sequence identity with SEQ ID NO: 61.

In some embodiments, the amino acid sequences of VH CDR1, 2 and 3 and VL CDR1, 2 and 3 of the antibody are: SEQ ID NOs: 66, 67, and 68, and SEQ ID NOs: 70, 71, and 72, and the VH sequence has at least 70% sequence identity with SEQ ID NO: 65, and the VL sequence has at least 70% sequence identity with SEQ ID NO: 69.

In some embodiments, the V region of the monoclonal antibody as described above can be linked to the C region of human antibody, and the human-mouse chimeric antibody can be obtained by expression in appropriate host cells.

In some embodiments, the framework region of the monoclonal antibody as described above may be or be replaced by a human-derived framework region (FR), such as only CDRs are retained while others are human-derived structures.

In some embodiments, non-human monoclonal antibody can be humanized through the following methods: (1) homologous substitution, which is conducted by replacing the corresponding non-human part with a human FR that has greater homology; (2) surface remodeling, which comprises a step of remodeling the amino acid residues on the surface of non-human CDRs and FRs, to make them resemble to the human CDRs or FRs forms; (3) compensatory mutation, which is conducted by adapting key amino acid residues to compensate for CDR transplantation; (4) site-specific conservative, where the monoclonal antibody is humanized using FR conserved sequences as templates, while retaining key amino acid residues in the variable region of non-human monoclonal antibody.

The present disclosure not only comprises a complete monoclonal antibody, but also comprises antibody fragments (antigen binding fragments) thereof with immune activity, such as Fab, Fab ', F (ab') ₂, Fd, single-stranded Fv or scFv, disulfide linked Fv, V-NAR domain, IgNar, intracellular antibody, IgGΔCH₂, mini-antibody, F(ab')₃, tetra-antibody, tri-antibody, bispecific antibody, single domain antibody, DVD-Ig, Fcab, mAb₂, (scFv)₂ or scFv-Fc, etc.

In some embodiments, the antibody of the present disclosure binds to TNFR2 derived from human or other primates, but does not bind to mouse TNFR2.

Monoclonal antibody and the fragment thereof can be prepared using various methods known to those skilled in the art. For example, monoclonal antibody can be prepared using the hybridoma method (proposed first by Kohler et al., Nature, 256:495 (1975)), or using the recombinant DNA method (US Patent No. 4816567). Monoclonal antibody can also be prepared by isolating from a phage antibody library, which is described in, such as Clackson et al., Nature, 352:624-628 (1991) and Marks et al., J. Mol. Biol, The 222:581-597 (1991).

### Encoding molecule of the monoclonal antibody or the fragment thereof, and expression vector and host cell containing the molecule

Nucleic acid molecules encoding the anti-TNFR2 monoclonal antibody or the fragment thereof are also provided herein. The sequences of these nucleic acid molecules can be obtained using conventional techniques, such as PCR amplification or genomic library screening. In addition, the encoding sequences of light and heavy chains can be fused together to form a single chain antibody. Further disclosed herein are expression vectors containing the aforementioned nucleic acid molecules, such as pcDNA3.4, pcDNA3.1, etc. Further disclosed are host cells transformed by the aforementioned expression vectors, which can be, for example, CHO cells, COS cells, etc.

Once the relevant sequence is obtained, recombination can be used to obtain it in large quantities. This is usually done by cloning it into a vector, then transferred into cells, and then isolating the relevant sequences from the proliferating host cells using conventional methods.

In addition, the relevant sequences can also be obtained by artificial synthesis methods, especially those with short lengths. Usually, long sequences can be obtained by synthesizing multiple small fragments first and then connecting them.

At present, the nucleic acid sequence encoding the monoclonal antibody (or the fragment, or derivative thereof) described herein can be obtained entirely through chemical synthesis. Then, the sequence can be introduced into various existing molecules (or vectors) and cells known in this field. In addition, mutations can be introduced into the protein sequence described herein through chemical synthesis.

Vectors containing the aforementioned encoding sequences and appropriate promoters or control sequences are also involved herein. These vectors can be used to transform appropriate host cells to express proteins. The host cells can be prokaryotic cells, such as bacterial cells; or lower level eukaryotic cells, such as yeast cells; or higher level eukaryotic cells, such as mammalian cells, such as CHO cells, COS cells, etc. Mammalian cell lines are typically used as host cells for expressing eukaryotic derived polypeptides. The proliferation of mammalian cells in culture is well-known in this field. See Organizational Cultivation, Academic Press, edited by Kruse and Patterson (1973), which is incorporated herein by reference. Preferred mammalian cells are commercially available infinitely proliferative cell lines. These infinitely proliferative cell lines incldue, but are not limited to, Chinese hamster ovary (CHO) cells, Vero cells, Hela cells, baby hamster kidney (BHK) cells, monkey kidney cells (COS), human hepatocellular carcinoma cells (such as Hep G2), and many other cell lines. They provide post-translational modifications for protein molecules, including correct folding, correct formation of disulfide bonds, and glycosylation at the correct sites.

A hybridoma cell line capable of producing the monoclonal antibody herein is also provided herein. After obtaining the hybridoma that produces the monoclonal antibody herein, those skilled in the art can easily obtain the structure of the antibody described herein (such as the heavy chain variable region and light chain variable region of the antibody), and then prepare the monoclonal antibody.

There are many methods for transforming host cells by expression vectors, and the transformation method depends on the host to be transformed. The method of introducing heterologous polynucleotides into mammalian cells is known in the art, which comprises glucan-mediated transfection, calcium phosphate precipitation, Polybrene (1,5-dimethyl-1,5-diazoundecylene polymethyl bromide) mediated transfection, protoplast fusion, electroporation, liposome mediated transfection, and direct microinjection of DNA into the nucleus. Herein, the preferred methods are electroporation or liposome mediated methods. For example, Invitrogen's liposome assay kit can be used to transfect host cells such as COS and CHO cells.

The transformed host cells can be cultivated under conditions suitable for the expression of monoclonal antibody herein. Then the monoclonal antibody can be purified by conventional purification steps of immunoglobulin, such as protein A-Sepharose, hydroxyapatite chromatography, gel electrophoresis, dialysis, ion exchange chromatography, hydrophobic chromatography, molecular sieve chromatography or affinity chromatography, which are well known to those skilled in the art.

### Identification and purification of the monoclonal antibody

The obtained monoclonal antibody can be identified using conventional methods. For example, the binding specificity of monoclonal antibody can be determined by immunoprecipitation or *in vitro* binding assays (such as radioimmunoassay (RIA) or enzyme-linked immunosorbent assay (ELISA)). The binding affinity of monoclonal antibody can be determined by, for example, Scatchard analysis (Munson et al., Anal Biochem, 107: 220 (1980)).

The monoclonal antibody herein can be expressed in cells, on cell membranes, or secreted outside cells. If necessary, the recombinant protein can be separated and purified through various separation methods utilizing its physical, chemical, and other properties. These methods are well-known to those skilled in the art. Examples of these methods include but are not limited to: conventional renaturation treatment, protein precipitation treatment (salting out method), centrifugation, osmotic bacteria breaking, ultrasonic treatment, ultracentrifugation, molecular sieve chromatography (gel filtration), adsorption chromatography, ion exchange chromatography, high-performance liquid chromatography (HPLC) and other various liquid chromatography technologies and the combination of these methods.

### Conjugate, chimeric antigen receptor, and immune cell expressing the chimeric antigen receptor

The present application also provides a conjugate and chimeric antigen receptor containing the antibody or antigen binding fragment thereof, , and immune cell expressing the chimeric antigen receptor. Due to the specificity of the antibody or the antigen binding fragment thereof to TNF2, the conjugates, chimeric antigen receptors, and immune cells expressing the chimeric antigen receptors can target cells or tissues expressing or overexpressing TNF2. Moreover, due to the inherent biological activity (such as immunomodulatory activity) of the antibody or the antigen binding fragment thereof of this application, the antibody or antigen binding fragment portion in the conjugate, chimeric antigen receptor, and immune cell expressing the chimeric antigen receptor can also play corresponding roles.

In some embodiments, the antibody conjugate may comprise: the antibody or the antigen binding fragment thereof described herein; a coupling moiety, such as a drug, toxin, cytokine, radioactive isotope, or enzyme; and optionally, linkers. In some embodiments, the coupling is achieved through enzymatic coupling, chemical coupling, fusion, and other methods. Linkers can be cleavable linkers that promote the release of coupling moiety in cells, such as acid-unstable linkers, peptidase-sensitive linkers, light-unstable linkers, dimethyl linkers, and linkers containing disulfides.

In some embodiments, the coupling moiety connected to the antibody may be, for example, a bioactive agent, which can be any synthetic, recombinant, or naturally occurring substance that enhances and/or regulates the desired biological action. In some embodiments, the effects that may be provided by the bioactive agent include but are not limited to: regulating, stimulating, and/or inhibiting growth signals; regulating, stimulating, and/or inhibiting anti-apoptotic signals; regulating, stimulating, and/or inhibiting apoptosis or necrosis signals; regulating, stimulating, and/or inhibiting the ADCC cascade; regulating, stimulating, and/or inhibiting the CDC cascade, etc. For example, it can be a chemotherapy agent, toxin, cytokine, isotope, enzyme, etc.

The present application also provides a chimeric antigen receptor (CAR) comprising an extracellular domain and an intracellular domain, wherein the extracellular domain comprises the antibody or the antigen binding fragment thereof of the present application. In some embodiments, the CAR of the present application further comprises a transmembrane domain connecting the extracellular domain and the intracellular domain, such as the transmembrane portion of CD28. The CAR for this application may be the first, second, third, or fourth generation CAR.

In some embodiments, the antibody or the antigen binding fragment thereof in the CAR of the present application is scFv or VH sdAb, or composed of scFv or VH sdAb. In some embodiments, the intracellular domain of CAR comprises those that simulate or approximate signals pa through natural antigen receptors, signals pass through the combination of such receptors and co-stimulatory receptors, and/or signals pass through individual co-stimulatory receptors.

In some embodiments, the intracellular domain comprises one or more co-stimulatory domains and activation domains, such as one or more selected from the group consisting of ITAM domain, CD3ζ, CD28, 4-1BB, OX40, CD27, ICOS, and a combination thereof, such as (CD28+CD3ζ), (CD28+CD27+CD3ζ), (CD28+OX40+CD3ζ), (CD28+4-1BB+CD3ζ), (CD28+CD27+OX40+CD3ζ), (CD28+4-1BB+CD27+CD3ζ), (CD28+4-1BB+OX40+CD3ζ), (4-1BB+CD3ζ), (4-1BB+OX40+CD3ζ), (4-1BB+CD27+CD3ζ), (CD27+CD3ζ), (CD27+OX40+CD3ζ), (CD28Δ+CD3ζ), (CD28Δ+CD27+CD3ζ), (CD28Δ+ OX40+CD3ζ), (CD28Δ+4-1BB+CD3ζ), (CD28Δ+4-1BB+OX40+CD3ζ), (CD28Δ+CD27+OX40+CD3ζ), (CD28Δ+4-1BB+CD27+CD3ζ), (4-1BB+ICOS+CD3ζ), (CD28+ICOS+CD3ζ), (ICOS+CD3ζ).

In some embodiments, the extracellular domain of CAR further comprises a hinge region, for example the hinge region originats from IgG hinge or CD8α/CD28 extracellular domain, such as selected from: IgG4 Fc Δ EQ, IgG4 Fc Δ Q, (t-12AA+t-20AA), mKate, phiLov, dsRed, Venus, eGFP, CH3 HA, (CD8α+t-20AA, dual t-20AA, (t-20AA+CD8α), (CD8α+Leucine zipper Basep1), (CD8α+Leucine zipper Acid1), 2D3, CD8α or IgG4 Fc.

The present application also provides a transformed immune cell that expresses the chimeric antigen receptor of the present application. For example, the immune cell is selected from T cell, such as αβ T cells, γδ T cell, NK T cell or T cell derived from pluripotent cell. The immune cells can be derived from an individual to be treated or from other sources. The transformed (optionally amplified) immune cells can be administrated to the individual to be treated, for example, for adoptive therapy.

In addition, the present application also comprises CAR constructs, expression vectors, preparation methods, and the applications for transforming the immune cells.

### Pharmaceutical composition

A pharmaceutical composition is also provided herein, which comprises: pharmaceutically effective amounts of the monoclonal antibody herein, the immunoconjugate thereof or the chimeric antigen receptor (CAR) thereof or the CAR transformed immune cells; and pharmaceutically acceptable carriers.

The term "pharmaceutically acceptable" used herein refers to the absence of adverse, allergic, or other adverse reactions when the molecular and composition thereof are appropriately administered to animals or humans. The "pharmaceutically acceptable carrier" used herein should be compatible with the active substance in this article, that is, it can be mixed without significantly reducing the effectiveness of the pharmaceutical composition under normal circumstances. These carriers are well-known to those skilled in this field. A thorough discussion on pharmaceutically acceptable carriers can be found in Remington's Pharmaceutical Sciences, Mack Pub. Co., N.J. 1991.

This type of carrier include (but is not limited to): saline, buffer, glucose, water, glycerol, ethanol, adjuvants, and the combinations thereof. In addition, thees carriers may also comprise auxiliary substances, such as wetting agent or emulsifier, pH buffers, etc.

The composition described herein can be administered orally, intravenously, intramuscularly, or subcutaneously; preferably, orally or intravenously. The pharmaceutical composition described herein can be made into various dosage forms according to needs, and the physician can determine the beneficial dosage for patients based on factors such as patient type, age, weight, approximate disease condition, and administration method, etc.

When a pharmaceutical combination is used, a safe and effective amount of monoclonal antibody or immune conjugates is administrated to mammals, wherein the safe and effective amount is usually about 0.1 micrograms to 5 milligrams per kilogram of body weight, and in most cases does not exceed about 3 milligrams per kilogram of body weight, preferably about 1-10 micrograms per kilogram to about 1 milligram per kilogram of body weight. Of course, when determining the specific dosage, some factors should also be considered, such as the route of administration and the patient's health status, which are within the skill scope of skilled physicians.

As used herein, the term "unit dosage form" refers to the dosage form required for single administration of the composition of the present disclosure for the convenience of administration, including but not limited to various solid agents (such as tablets), liquid agents, capsules, and sustained-release agents.

In another preferred embodiment disclosed herein, the composition is a unit dosage form or multiple dosages form, and the content of the active substance is 0.01-2000mg/dose, preferably 0.1-1500mg/dose, and more preferably 1-1000mg/dose. In another preferred embodiment disclosed herein, 1-6 doses, preferably 1-3 doses, most preferably 1 dose of the disclosed composition are administered daily.

According to the functions and effects of the active substances described iherein in specific environments, the active substances can be classified as "immunoactive" and "immunosuppressive" active substances. As used herein, the term "immunoactive" active substance refers to a substance that can enhance immune response or function, which can be achieved by, for example, inhibiting the proliferation and/or activity of regulatory T cells (Tregs) and/or promoting the proliferation and/or activity of effector T cells (Teff). The "immunosuppressive" active substance refers to a substance that can reduce immune response or function, which can be achieved, for example, by inhibiting the proliferation and/or activity of effector T cells (Teff) and/or increasing the proliferation and/or activity of regulatory T cells (Treg). It should be understood that the immunosuppressive and immunoactive properties of the active substance in the present application can vary with the environment. For example, in a tumor microenvironment, it may be an immunoactive active substance, while in an allergic microenvironment, it may be an immunosuppressive active substance.

In some embodiments, the active substance described herein can play a positive role of antagonizing TNFR2 immunoregulatory in the microenvironment of tumor or infectious diseases. The immunoactive monoclonal antibodies or immune conjugates, compositions, and other active substances herein can inhibit the proliferation and/or activity of regulatory T cells (Tregs), promote the proliferation and/or activity of effector T cells (Teff), inhibit tumor cells, promote inflammation, and inhibit infection. For example, the monoclonal antibody or the antigen binding fragment thereof can be used to enhance immune response, anti-tumor (preferably TNFR2 expression or overexpression types), and anti-infection (such as inducing pro-inflammatory response). In some embodiments, the immunoactive substance herein can be used to prevent and/or treat tumors, infections or infectious diseases. The active substance herein can be used to treat various types of tumors, including but not limited to: adenocarcinoma, leukemia, lymphoma, melanoma, sarcoma; tumor tissues from the adrenal gland, gallbladder, bone, bone marrow, brain, breast, bile duct, gastrointestinal tract, heart, kidney, liver, lungs, muscles, ovaries, pancreas, parathyroid gland, penis, prostate, skin, salivary gland, spleen, testes, thymus, thyroid or uterus; tumors of the central nervous system, such as glioblastoma or astrocytoma; eye tumors (such as basal cell carcinoma, squamous cell carcinoma, or melanoma), endocrine gland tumors, neuroendocrine system tumors, gastrointestinal pancreatic endocrine system tumors, reproductive system tumors, or head and neck tumors.

In some embodiments, the immunosuppressive monoclonal antibody or the fragment or composition thereof can promote the proliferation and/or activity of regulatory T cells (Tregs), inhibit the proliferation and/or activity of effector T cells (Teff), downregulate immune activity, and inhibit inflammatory responses. In some embodiments, the immunosuppressive active substance described herein can be used to suppress immune responses, resist autoimmune diseases, resist cytokine storms, and resist allergic diseases. For example, the autoimmune diseases include but are not limited to: Th2 lymphocyte related diseases (such as atopic dermatitis, atopic asthma, nasal conjunctivitis, allergic rhinitis, Euclidean syndrome, systemic sclerosis, graft-versus-host disease); Th1 lymphocyte related diseases (such as rheumatoid arthritis, multiple sclerosis, psoriasis, Schogren's syndrome, Hashimoto's thyroiditis, Grave's disease, primary biliary cirrhosis, Wegener's granulomatosis and tuberculosis); Activated B lymphocyte related diseases (such as systemic lupus erythematosus, nephritis syndrome, rheumatoid arthritis, and type I diabetes).

### Detection reagent kit

A reagent kit for detecting TNFR2, which contains the anti-TNFR2 monoclonal antibody or the fragment thereof and the immunoconjugate as described herein. The kit described herein can be used to detect the presence or content of TNFR2 in biological samples. The detection method comprises the steps of: (a) contacting the sample with the anti-TNFR2 monoclonal antibody or immunoconjugate in the kit described herein; (b) detect whether an antigen-antibody complex is formed, wherein the formation of the complex indicates the presence of TNFR2 in the sample, or the amount of the formed antigen-antibody complex determined by quantitative detection indicates the content of TNFR2 in the sample. The sample can be pre-treated or not, such as being extracted, purified, or concentrated.

The reagent kit comprises a container and the monoclonal antibody described herein located within the container, or a detection plate containing the monoclonal antibody, as well as a user manual. The reagent kit may further comprise other reagents required for detection, such as buffer, indicator, etc. Those skilled in this field can adjust the contents of the reagent kit according to specific needs.

### Examples

The present invention will be further elaborated with specific examples. It should be understood that these examples are only used to illustrate the present invention and not to limit the scope of the present invention. Technicians in this field may make appropriate modifications and variations to the present invention, all of which are within the scope of the present invention.

For experimental methods in the following examples that do not specify specific conditions, conventional methods in this field can be used, such as referring to the Molecular Cloning Experiment Guidelines (Third Edition, New York: Cold Spring Harbor Laboratory Press, 1989) or following the conditions recommended by the supplier. The DNA sequencing method is a conventional method in this field and can also be tested by commercial company.

Unless otherwise specified, percentages and portions are calculated by weight. Unless otherwise defined, all professional and scientific terms used herein have the same meanings as those familiar to those skilled in the field. In addition, any methods and materials similar or equal to the recorded content can be applied to the methods of the present invention. The preferred implementation methods and materials described in the article are only for demonstration purposes.

### Example 1: Production of anti-human TNFR2 mouse monoclonal antibody

### 1.1 Immunization

10 Balb/c mice (6-8 weeks old, weighing around 18g, female, purchased from the Experimental Animal Management Department of the Institute of Family Planning) were multi-point immunized via intraperitoneal and subcutaneous routes (injection volume of 50 µl per point) by 50 µg recombinant TNFR2-FC (Shanghai Saijin Biopharmaceutical Co., Ltd., Qiangke^{®}, catalog number A100114U) emulsificated in the presence of complete Freund's adjuvant (CFA) at 100 µl protein and 100 µl CFA. At the second, third, fourth, and fifth weeks, in the presence of incomplete Freund's adjuvant, the same procedure was conducted at 100 µl protein and 100 µl IFA to emulsificate and immunize.

### 1.2 Fusion

The spleen cells and lymphocytes of immunized mice were fused with mouse myeloma cells (purchased from the cell bank of the Chinese Academy of Sciences, catalog number: TCM18) by the following steps:
After 72 hours of booster (at the sixth week, subcutaneous injection of 25µg TNFR2-Fc fusion protein), the mice were eyeball-bloodletted, neck pulled and sterilized. The spleen was aseptically taken out, the surrounding connective tissues were stripped off, and the spleen was subject to washing. The spleen cells were cut and grinded, dispersed, filtered, centrifuged, resuspended, and counted. The myeloma cells were passaged, after 24 hours, mixed well with the mouse spleen cells, and centrifuged, all cell supernatants were discarded, and the cells were preheated in a 37 °C water bath. 1ml PEG preheated to 37 °C were added into a centrifuge tube within 60 seconds, gently stirred for 1 minute, and added with the culture medium, put stand for 10 minutes and centrifuged. The supernatant was discarded, and the cells were resuspended in 20% FBS IMDM medium (containing 1x HAT (Sigma, H0262-10VL) and 1x double antibiotics (Gibco, 15140122)), divided into 25 96-well cell culture plates and incubated (200 µl/well, 5% CO₂, 37 °C).

### 1.3 Hybridoma screening

On the 5th to 10th day after fusion, according to the cell growth density, the entire medium was replaced with IMDM medium containing 10% FBS (1 × HAT and 1 × double antibiotics). On the 10th to 14th day after fusion, ELISA detection was performed based on cell growth density. The positive well cells were subjected to the first and second subcloning.

The ELISA detection steps are as follows: The ELISA plate was coated with TNFR2-His (Sino Biological, 10417-H08H, Assessment# NP:001057.1.Met1-Asp257, with multi-histidine tag expressed on C-terminus), then the ELISA plate was blocked, and incubated with the hybridoma supernatant (which was added to the ELISA plate at 100 µl/well, sealed by the plate membrane and put stand at 37 °C for 1 hour), then incubated with with goat anti-mouse HRP secondary antibody (Jackson Immuno Research, 115-035-164; which was diluted with a binding solution of 1:3000, added to the ELISA plate at 100 µl/well, incubated at 37 °C for 1 hour). After TMB development, the detection was performed using microplate reader: dual wavelength 450nm/655nm detection, and results were calculated by OD₄₅₀-OD₆₅₅.

### 1.4 Preparation of mouse monoclonal antibody

After 2 rounds of subcloning, 10 strains of antibodies binding to hTNFR2 were screened, which are 16D2, 27F6, 29A1, 50E7, 7F2, 11G2, 12E12, 13F5, 18A12, and 33C12, respectively. The information is shown in the table below:

**Table 1. Clone Information**

| Clone | Heavy chain | Light chain |
|---|---|---|
| 16D2 | IgG1 | κ |
| 27F6 | IgG1 | κ |
| 29A1 | IgG1 | κ |
| 50E7 | IgG1 | κ |
| 7F2 | IgG1 | κ |

| Clone | Heavy chain | Light chain |
|---|---|---|
| 11G2 | IgG2b | κ |
| 12E12 | IgG1 | λ |
| 13F5 | IgG1 | λ |
| 18A2 | NA | κ |
| 33C12 | LgG1 | κ |

### 1.5 Identification of Mouse Monoclonal Antibody

### 1.5.1 Binding of anti-human TNFR2 mouse monoclonal antibody to recombinant human TNFR2 antigen

The binding of 8 strains of TNFR2 monoclonal antibodies to recombinant human TNFR2 antigen (Kangdai, batch number A100114U) was demonstrated using standard ELISA method. The specific steps are as follows:
The ELISA plate was coated with TNFR2 antigen (1µg/ml, 50µl/well) and blocked, incubated with gradient diluted TNFR2 monoclonal antibody (37 °C, 1h), then incubated with goat anti-mouse HRP secondary antibody (Jackson Immuno Research, 115-035-164; diluted with a binding solution of 1:3000, added to the ELISA plate at 100 µl/well). After TMB development, the detection was performed using microplate reader: dual wavelength 450nm/655nm detection, and results were calculated by OD₄₅₀-OD₆₅₅.

### Experimental results and analysis:

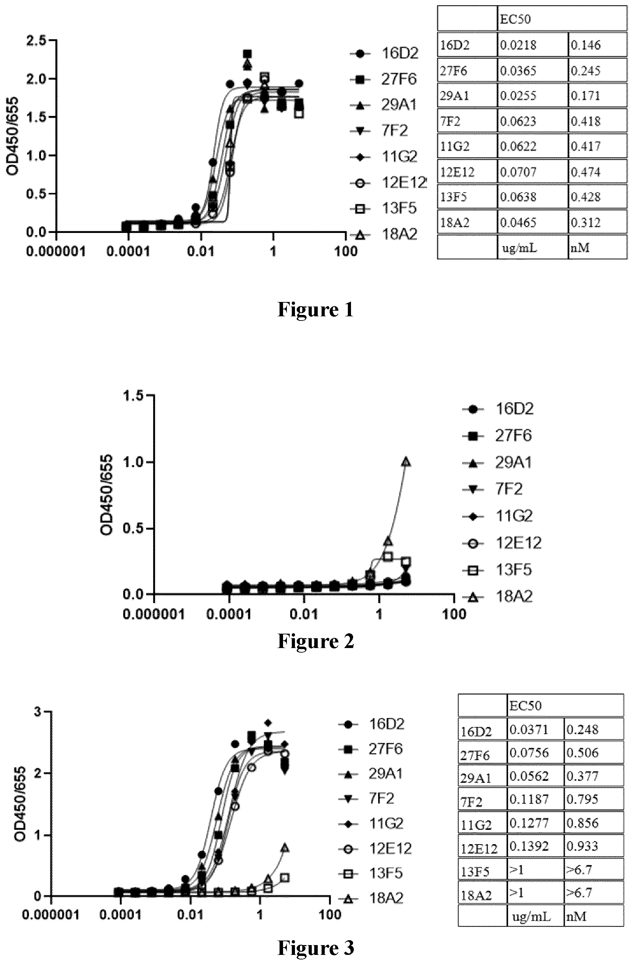

The ELISA results are shown in Figure 1. The results showed that all 8 strains of anti-human TNFR2 mouse monoclonal antibodies (clone numbers 16D2, 27F6, 29A1, 7F2, 11G2, 12E12, 13F5, 18A2) could specifically bind to human TNFR2 with high affinity.

The EC₅₀ of 7F2, 11G2, 12E12, 13F5, 18A2, 16D2, 27F6, and 29A1 binding to TNFR2 were 62.3ng/ml, 62.2ng/ml, 70.7ng/ml, 63.8ng/ml, 46.5ng/ml, 21.8ng/ml, 36.6ng/ml, and 25.5ng/ml, respectively.

### 1.5.2 Binding of anti-human TNFR2 mouse monoclonal antibody to recombinant mouse TNFR2 and cynomolgus monkey TNFR2

Using the same method as above, the TNFR2 antigens of cynomolgus monkey and mouse (Sino Biological, 90102-C02H and 50128-M08H) were coated separately. The anti-human TNFR2 mouse monoclonal antibodies to be tested were gradient diluted, then added with the anti-mouse Fab segment secondary antibody (Jackson Immuno Research, 515-035-072), and TMB development was performed. ELISA was used to detect the binding of 8 strains of mouse monoclonal antibodies to recombinant mouse TNFR2 (mTNFR2) and cynomolgus monkey TNFR2 (Cyno. TNFR2).

### Experimental results and analysis:

The results are shown in Figures 2 and 3, respectively. The results showed that anti-human TNFR2 mouse monoclonal antibodies 7F2, 11G2, 12E12, 13F5, 18A2, 27F6, and 29A1 did not bind to mouse TNFR2; 13F5 and 18A2 did not bind to cynomolgus monkey TNFR2. 7F2, 11G2, 12E12, 16D2, 27F6, and 29A1 could bind to cynomolgus monkey TNFR2, with EC₅₀ values of 118.7 ng/ml, 127.7 ng/ml, 139.2 ng/ml, 37.1 ng/ml, 75.6 ng/ml, and 56.2 ng/ml, respectively.

### 1.5.3 Binding of anti-human TNFR2 mouse monoclonal antibody to TNFR2-CHO cells

Purified anti-human TNFR2 mouse monoclonal antibodies were co-incubated with TNFR2 overexpressing CHO cell lines (obtained routinely as follows: PCDNA3.4-TNFR2 was transfected into CHO cells, and stable high expression TNFR2 cell lines were obtained through drug screening and monoclonal sorting), and then stained. The antibody binding intensity was analyzed by flow cytometry. The specific steps are as follows: TNFR2-CHO cells were counted, centrifuged, resuspended, then added to a 96-deep well plate, and the supernatant was discarded. The anti-TNFR2 antibodies to be tested were gradient diluted, added to the 96-deep well plate. After incubation and wash, the Anti-mouse-IgG-PE (Jackson Immuno Research, 115-116-071) was added. The samples were detected using Beckman CytoFLEX.

### Experimental results and analysis

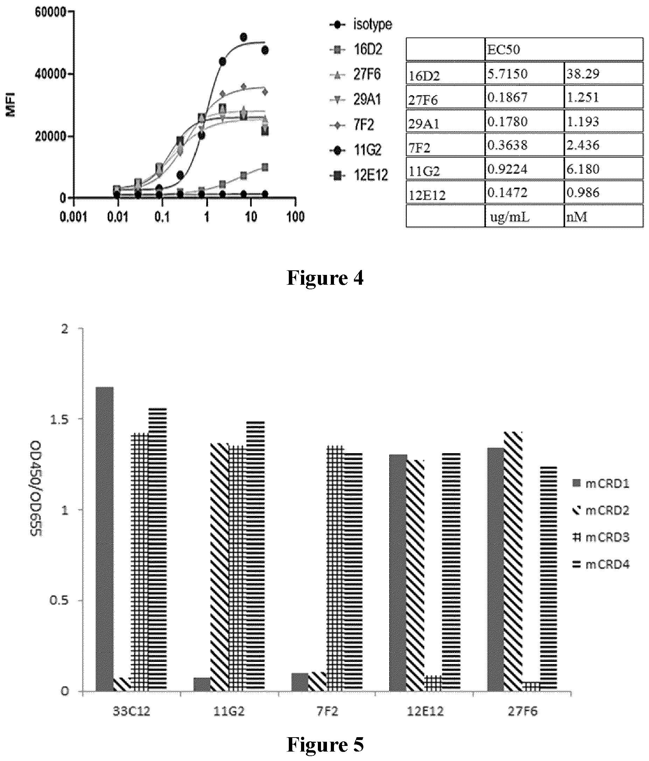

As shown in Figure 4, anti-human TNFR2 mouse monoclonal antibodyies 16D2, 27F6, 29A1, 7F2, 11G2, and 12E12 could all bind to TNFR2 on the cell membrane. The EC₅₀ values for binding to TNFR2 were 5715ng/ml, 186.7ng/ml, 178ng/ml, 363.8ng/ml, 922.4ng/ml, and 147.2ng/ml, respectively. The Isotype in the figure is a negative control for isotype antibody (mouse IgG1).

### 1.5.4 Analysis of binding epitopes of anti-human TNFR2 mouse monoclonal antibody to recombinant human TNFR2 antigen

The experimental results of 1.5.2 indicate that the TNFR2 mouse monoclonal antibody of the present invention has no binding activity to mouse TNFR2 antigen. To investigate the binding regions of TNFR2 mouse monoclonal antibody to human TNFR2, a series of TNFR2 mutants was constructed, in which the cysteine-rich domains (CRD) CRD1, CRD2, CRD3, and CRD4 of human TNFR2 were replaced by the corresponding regions of mouse (i.e., mCRD1, mCRD2, mCRD3, mCRD4). The mutant proteins were obtained through transient transfection and expression, and the binding activity with TNFR2 mouse monoclonal antibody was detected.

The experimental method is as follows: The pcDNA3.4 plasmids synthesized by gene synthesis were TNFR2-mCRD1, TNFR2-mCRD2, TNFR2-mCRD3, and TNFR2-mCRD4, respectively. Four mCRD proteins were expressed in 293F cells and purified by ProteinA. Four mCRDs were coated separately, and the ELISA plate was blocked, and gradient diluted TNFR2 mouse monoclonal antibodies were added for incubation. Then the goat anti-mouse HRP secondary antibody (Jackson Immuno Research, 115-035-164) was added for incubation, and TMB development was performed. The detection was performed using microplate reader: dual wavelength 450nm/655nm detection, and results were calculated by OD₄₅₀-OD₆₅₅.

FACS detection: the TNFR2-CHO cells were counted, centrifuged, resuspended, then added to a 96-deep well plate, and the supernatant discarded. Gradient diluted anti-TNFR2 mouse monoclonal antibodies were added to a 96-deep well plate for incubation. Then the plate was washed, and added with Anti-mouse-IgG-PE (Jackson Immuno Research, 115-116-071) for incubation. The samples were detected using Beckman CytoFLEX.

### Experimental results and analysis:

The results are shown in Figure 5. When a human TNFR2 antigen binding region CRD is replaced with a mouse mCRD, if it does not bind to TNFR2 monoclonal antibody, then that region is the antigen-binding region of TNFR2 monoclonal antibody to recombinant human TNFR2 antigen. The results showed that TNFR2 monoclonal antibody 7F2 bound to recombinant human TNFR2 antigens CRD1 and CRD2; 11G2 bound to CRD1; 12E12 bound to CRD3; 27F6 bound to CRD3; and 33C12 bound to CRD2.

### 1.6 Sequencing of heavy and light chain variable regions

Nine strains of hybridomas specifically binding to TNFR2 were screened in the early stage, with clone numbers 16D2, 27F6, 29A1, 7F2, 11G2, 12E12, 13F5, 18A2, and 33C12, respectively. By sequencing the IgG V region, the sequence information of the V region was successfully obtained, as shown in the table below:

**Table 2. V region sequences of 9 strains of TNFR2 mouse monoclonal antibodies.**

| **Clone** | **Description** | | **SEQ ID** | **Sequence** |
|---|---|---|---|---|
| 16D2 | VH | Full length | 1 | EVQLQQSEPELVKPGASVKIPCKASGYTFTDYNMDWVKQSHGKSL EWIGDINPNNGVTIYNQKFKGKATLTVDKSSSTAYMELRSLTSEDT AVYYCVILEYFDYWGQGTTLTVSS |
| | | CDR1 | 2 | DYNMD |
| | | CDR2 | 3 | DINPNNGVTIYNQKFKG |
| | | CDR3 | 4 | LEYFDY |
| | VL | Full length | 5 | DIVMTQSTSSMSVSLGDTVSITCHASQGISSNIGWLQQKPGKSFKGL IYHGTKLEDGVPSRFSGSGSGADYSLTISSLESEDFADYYCVQYAQF PRTFGGGTKLEIK |
| | | CDR1 | 6 | HASQGISSNIG |
| | | CDR2 | 7 | HGTKLED |
| | | CDR3 | 8 | VQYAQFPRT |
| 27F6 | VH | Full length | 9 | EVQLQQSGTVLARPGASVKMSCKTSGYTFTSYWMHWVKQRPGQG LEWIGTIYPGNSDTSYNQKFKGKAKLTAVTSASTAYMELNSLTNED SAVFYCTRENLLLRSYFFDYWGQGTTLTVSS |
| | | CDR1 | 10 | SYWMH |
| | | CDR2 | 11 | TIYPGNSDTSYNQKFKG |
| | | CDR3 | 12 | ENLLLRSYFFDY |
| | VL | Full length | 13 | DIVMTQSPASLAVSLGQRATISCKASQSVDYDGDSYLNWYQQKPG QPPKLLIYAASNLPSGIPARFSGSGSGTDFTLNIHPVEEEDAATYYCQ QSNEDPRTFGGGTKLEIK |
| | | CDR1 | 14 | KASQSVDYDGDSYLN |
| | | CDR2 | 15 | AASNLPS |
| | | CDR3 | 16 | QQSNEDPRT |
| 29A1 | VH | Full length | 17 | QVQLQQSGAEVVRPGASVTLSCKASGYTFSDYEMHWVKQTPVHG LEWIGAIDPETGDTAYNQKFKDKARVTADKSSSTAYMELRSLTSED SAVYYCTRDIHYYGSSLSYFDYWGQGTTLTVSS |
| | | CDR1 | 18 | DYEMH |
| | | CDR2 | 19 | AIDPETGDTAYNQKFKD |
| | | CDR3 | 20 | DIHYYGSSLSYFDY |
| | VL | Full length | 21 | DIVMTQSQKFMSTSVGDRVSVTCKASQNVGTNVAWYQQKPGQSP KVMIYSASYRYSGAPDRFTGSGSGTDFTLTVNNVQSEDLAEYFCQQ YKTYPYTFGGGTKLEIE |
| | | CDR1 | 22 | KASQNVGTNVA |
| | | CDR2 | 23 | SASYRYS |
| | | CDR3 | 24 | QQYKTYPYT |
| 7F2 | VH | Full length | 25 | QVTLKESGPGILQPSQTLSLTCSFSGFSLNTFGLGVGWIRQPSGKGL EWLTHIWWNDDKYYNPALKSRLTISRDTSENLVFLKIANVDTADTA TYYCARIEGLRRFDYWGQGTTLTVSS |
| | | CDR1 | 26 | TFGLGVG |
| | | CDR2 | 27 | HIWWNDDKYYNPALKS |
| | | CDR3 | 28 | IEGLRRFDY |
| | VL | Full length | 29 | DIQMTQSPSSLSASLGGKVTITC**KTSQDINKFIA**WYQHKPGKGPRL LIH**YTSTLQP**GIPSRFSGSGSGRDYSFSISDLEPEDIATYYC**LQYANL** **WA**FGGGTKLEIK |
| | | CDR1 | 30 | KTSQDIN KFIA |
| | | CDR2 | 31 | YTSTLQP |
| | | CDR3 | 32 | LQYANLWA |
| 11G2 | VH | Full length | 33 | QVHLQQSGPELVKPGAPVKLSCKASGYTFITYDINWVKQRPGQGLE WIGWIYPRDGNTQFNENFKGKATMSVDESSSTAYMEVHSLTSEDS AVYFCARGDRDYGYFDVWGTGTTVSVSS |
| | | CDR1 | 34 | TYDIN |
| | | CDR2 | 35 | WIYPRDGNTQFNENFKG |
| | | CDR3 | 36 | GDRDYGYFDV |
| | VL | Full length | 37 | DIQMTPTTSSLSVSLGDRVTISYRASQDISNYLNWYQQKPDGTVKL LIYFKSKLHSGVPSRFSGSGSGTDFSLTIANLEQEDIATYFCQQGNT LPLTFGVGTRLALK |
| | | CDR1 | 38 | RASQDISNYLN |
| | | CDR2 | 39 | FKSKLHS |
| | | CDR3 | 40 | QQGNTLPLT |
| 12E12 | VH | Full length | 41 | EVQLQQSGAELVKPGASVKLSCTVSGFNIKDFYIHWVKQRPEQGLE WIGRVDPEDGNTEYAPNFQDRATMTTDTSSNTAYLQLSSLTSVDTA VYYCSTWDGFSYFDYWGLGTTLTVSS |
| | | CDR1 | 42 | DFYIH |
| | | CDR2 | 43 | RVDPEDGNTEYAPNFQD |
| | | CDR3 | 44 | WDGFSYFDY |
| | VL | Full length | 45 | QAVVTQESALTTSPGETVTLTCRSSTGPVTTNNYANWVQEKPDHLF TGLIGGTNDRAPGVPARFSGSLIGDKTALTITGTQTEDEAVYFCALW YRNHWVFGGGTKLTVL |
| | | CDR1 | 46 | RSSTGPVTTNNYAN |
| | | CDR2 | 47 | GTNDRAP |
| | | CDR3 | 48 | ALWYRNHWV |
| 13F5 | VH | Full length | 49 | QVQLQQSGAELVRPGTSVKMSCKASGYTFTNYWIGWTKQRPGHG LEWIGDIFPGGDFTNYNEKFKGKATLTADKSSSTAYLQFSSLTSEDS AIYYCSRRRFTTIVAPGFDYWGQGTTLTVSS |
| | | CDR1 | 50 | NYWIG |
| | | CDR2 | 51 | DIFPGGDFTNYNEKFKG |
| | | CDR3 | 52 | RRFTTIVAPGFDY |
| | VL | Full length | 53 | QLVLTQSSSASFSLGASAKLTCTLSSQHSMYTIEWYQQQPLKPPKF VMELKKDGSHSTGDGIPDRFSGSSSGADRYLNISNIQPEDEAIYICG VGDTIKEQFVYVFGGGTEVTVL |
| | | CDR1 | 54 | TLSSQHSMYTIE |
| | | CDR2 | 55 | GSHSTGD |
| | | CDR3 | 56 | GVGDTIKEQFVYV |
| 18A2 | VH | Full length | 57 | DVQLQESGPGLVKPSQSLSLTCSVTGYSITSGYYWNWIRQFPGNKL EWMGYISYDGSNNYNPSLKKRISITRDTSKNQFFLKLNSVTTEDTA TYYCTGGSGTSWGQGTLVTVSA |
| | | CDR1 | 58 | SGYYWN |
| | | CDR2 | 59 | YISYDGSNNY NPSLKK |
| | | CDR3 | 60 | GSGTS |
| | VL | Full length | 61 | DIVMTQSHKFMSTSVGDRVSITCKASQDVTTGIAWYQQKPGQSPK LLIYSASNRFTGVPDRFTGSGSGTDFTFTISSVQAEDLAVYFCQQHY STPFTFGSGTKLEIQ |
| | | CDR1 | 62 | KASQDVTTGIA |
| | | CDR2 | 63 | SASNRFT |
| | | CDR3 | 64 | QQHYSTPFT |
| 33C12 | VH | Full length | 65 | DVKLVESGGGLVKPGGSLKLSCAASGFTFSDYTMSWVRQTPEKRL EWVATISTGGSYTYYPDSVKGRFTISRDNAKNTLYLQMSSLKSEDT AMYYCTRDGGYDGDYFDYWGQGTTLTVSS |
| | | CDR1 | 66 | DYTMS |
| | | CDR2 | 67 | TISTGGSYTYY PDSVKG |
| | | CDR3 | 68 | DGGYDGDYFDY |
| | VL | Full length | 69 | ENVLTQSPAIMSASPGEKVTMTCRASSSVSSSYLHWYQQKSGASPK LWIYRTSNLASGVPARFSGSGSGTSYSLTISSVEAEDAATYYCQQYS GYPLTFGAGTRLELK |
| | | CDR1 | 70 | RASSSVSSSYLH |
| | | CDR2 | 71 | RTSNLAS |
| | | CDR3 | 72 | QQYSGYPLT |

### Example 2. Preparation of human-mouse chimeric monoclonal antibody

### 2.1 Expression and purification of human-mouse chimeric monoclonal antibody

The 293F cells, culture medium, and transfection reagents used in this experiment were all purchased from Sino Biological, Inc. The vectors of human-mouse chimeric monoclonal antibody were transiently transfected into 293F cells by sinosection transfection reagent, then the cells were cultured and harvested. Protein purification was performed using proteinA beads, and the purified protein was analyzed by SDS-PAGE. The reduced and non-reduced bands of each antibody were normal in size.

### Example 3: Binding of Human-mouse Chimeric Monoclonal Antibody to TNFR2

### 3.1 ELISA test on the in vitro binding activity of human-mouse chimeric monoclonal TNFR2 antibody to human TNFR2

To determine whether the human-mouse chimeric TNFR2 monoclonal antibody of the present invention can bind to recombinant human TNFR2 protein, *in vitro* ELISA test was conducted. Similar to the method described above, The plate was coated with TNFR2-His (Sino Biological, 10417-H08H, Assessment# NP_001057.1.Met1-Asp257, with multi-histidine tag expressed on C-terminus) antigens, and blocked with BSA, then added with gradient diluted human-mouse chimeric TNFR2 monoclonal antibodies for incubation. Then, secondary antibody Ap-anit-human IgG Fc (JacksonImmuno Research, 109-055-098) was added for incubation After TMB development, ELISA reading was performed.

### Experimental results and analysis:

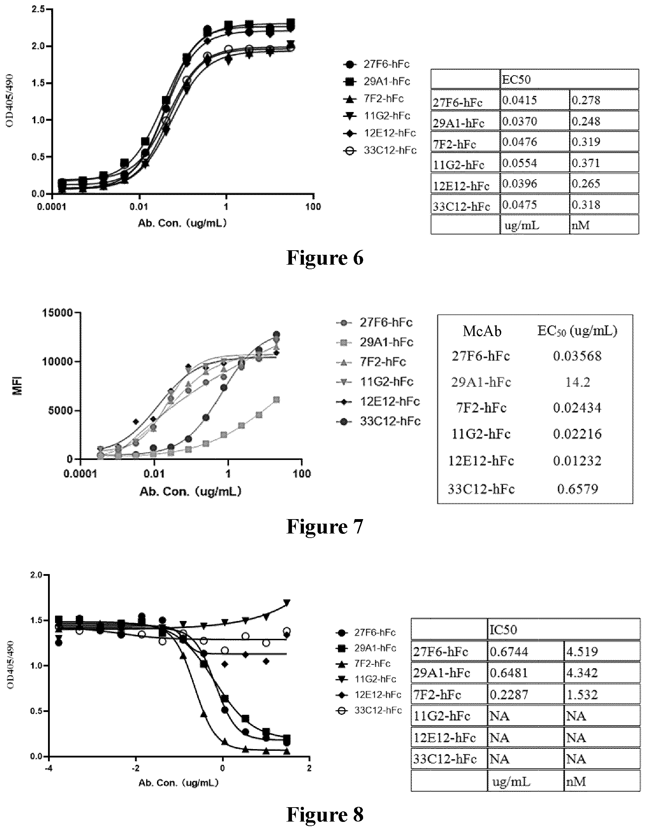

The ELISA results are shown in Figure 6. The results showed that the selected six strains ofhuman-mouse chimeric monoclonal antibodies (27F6-hFc, 29A1 hFc, 7F2 hFc, 11G2 hFc, 12E12-hFc, 33C12-hFc) with better binding affinity to cynomolgus monkey TNFR2 could specifically bind to human TNFR2, with binding affinity EC₅₀ values of 0.278 nM, 0.248 nM, 0.319 nM, 0.371 nM, 0.265 nM, and 0.318 nM, respectively.

### 3.2 Flow cytometry study on the binding of human-mouse chimeric monoclonal antibody to TNFR2 protein on cell membrane

To determine whether the human-mouse chimeric TNFR2 monoclonal antibody of the present invention can bind to the TNFR2 protein on the cell membrane, *in vitro* test was conducted.

Purified human-mouse monoclonal antibodies against TNFR2 were co-cultured with TNFR2 positive Jurkat cells (Jurkat cells were transfected with pcDNA3.4-TNFR2 plasmid using conventional methods, and after screening, stable and highly expressed TNFR2 Jurkat cell lines were obtained) and stained. The antibody binding intensity was analyzed by flow cytometry. The specific steps are as follows: Jurkat-TNFR2 was counted, centrifuged, resuspended, then added to a 96-deep well plate, and the supernatant was discarded. The anti-TNFR2 antibody was gradient diluted, and added to the 96-deep well plate, After incubation and wash, the secondary antibody FITC-anti-human IgG (Abcam: 6854) was added, and the samples were detected using Beckman CytoFLEX.

### Experimental results and analysis:

The results are shown in Figure 7. The results showed that the human-mouse chimeric TNFR2 monoclonal antibody 29A1-hFc had poor binding activity with TNFR2 on the cell membrane, while the other antibody 27F6-hFc, 7F2-hFc, 11G2-hFc, and 12E12-hFc had strong binding activity, followed by 33C12-hFc. The EC₅₀ values of these five antibodies were 0.036 µg/mL, 0.024µg/mL, 0.022µg/mL, 0.012µg/mL, 0.66µg/mL, respectively.

The results indicate that the antibody of the present invention can not only bind to solid-phase antigen (ELISA method), but also to TNFR2 on the cell membrane.

### Example 4. Inhibition of human-mouse chimeric TNFR2 monoclonal antibody binding to recombinant human TNFR2 and TNFα

To determine whether the human-mouse chimeric TNFR2 monoclonal antibody can inhibit the binding of TNFα to TNFR2, an *in vitro* test was conducted.

The TNFα used in this experiment is a fusion protein labeled with biotin, which has the activity of binding to TNFR2. The acquisition of the fusion protein can be found in Example 1 of the PCT application WO2020/118605A1 Briefly, the gene encoding the fusion protein was connected with the gene encoding TNFα through a two-step polymerase chain reaction (PCR) method. The complete cDNA of the heavy and light chains of the fusion protein were synthesized by GenScrip (USA) company, and cloned into the pUC57 vector, respectively. The cDNA of human TNFα was purchased from OpenBiosystems (USA).

To investigate whether the antibody in this experiment blocks the binding of TNFα to TNFR2, ELISA and FACS were conducted, respectively:

### 4.1 ELISA test

The ELISA plate was coated with TNFR2-His antigen (Sino Biological, 10417-H08H, Assessment# NP_001057.1. Met1-Asp257, with multi-histidine tags expressed on C-terminus) and blocked, added with the gradient diluted TNFR2 monoclonal antibodies and biotin-labeled TNFα fusion protein of fixed concentration for incubation, then added with the secondary antibody Ap-Strepavidin (Herobio, 510401) for incubation. After development, the detection was performed using microplate reader.

### Experimental results and analysis:

The results are shown in Figure 8. The results showed that three chimeric TNFR2 monoclonal antibodies (27F6-hFc, 29A1 hFc, 7F2 hFc) could specifically block the binding of TNF to soluble TNFR2. The IC₅₀ values were 0.6441 µg/mL, 0.6421 µg/mL, 0.2251 µg/mL, respectively.

### 4.2 FACS test

Purified anti-human TNFR2 human-mouse monoclonal antibodies were co-cultured with TNFR2 positive cells and stained. The antibody binding intensity was analyzed by flow cytometry. The specific steps are as follows: cells were counted, centrifugated, resuspended, then added to a 96-deep well plate, and the supernatant was discarded. Gradient diluted anti-TNFR2 antibodies and TNFα fusion protein of fixed concentration were added. After incubation and wash, the secondary antibody PE-streptavidin (Biogen, 405203) was added for incubation, and the samples were detected using Beckman CytoFLEX.

### Experimental results and analysis:

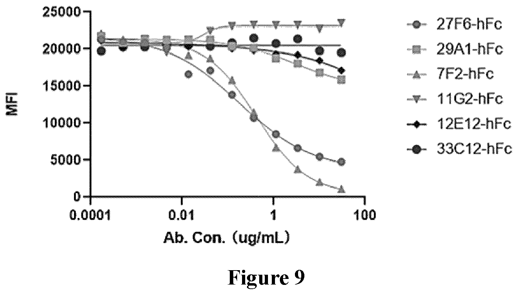

The experimental results are shown in Figure 9. The results showed that two chimeric TNFR2 monoclonal antibodies (27F6-hFc, 7F2 hFc) could specifically block TNFα binding to TNFR2 on the cell membrane. The IC50 values were 0.1786 µg/mL, 0.4166µg/mL, respectively.

**In summary,** the human-mouse chimeric TNFR2 monoclonal antibody 7F2 and 27F6 of the present invention can effectively block TNFα binding to TNFR2, and the activity of 7F2 is superior to that of 27F6, while other antibodies 29A1, 11G2, 12E12, and 33C12 have no blocking activity. This result suggests that these antibodies may act on TNFR2 through different sites and mechanisms.

### Example 5: Binding of Human-mouse chimeric TNFR2 monoclonal antibody to human TNFR2 on T cell surface

To determine whether the human-mouse chimeric TNFR2 monoclonal antibody can bind to the TNFR2 antigen on the surface of human T cells, FACS test was conducted *in vitro.*

Purified anti-human TNFR2 human-mouse chimeric monoclonal antibodies were co-cultured with PBMC cells (Oricells) and stained. The binding of the antibody was analyzed by flow cytometry. The specific steps are as follows: PBMC cells (Oricells) were counted, centrifuged, resuspended, then added to a 96-deep well plate, and the supernatant was discarded. Diluted anti-TNFR2 monoclonal antibodies were added. After incubation and wash, the secondary antibody Cy5-AffiniPure F(ab') Fragment Goat Anti-Human IgG, Fcy fragment specific (JacksonImmuno Research, 109-176-098) and FITC-anti-human CD3 antibody (BD Bioscience, 556611) were added, the the samples were detected using Beckman CytoFLEX.

### Experimental results and analysis:

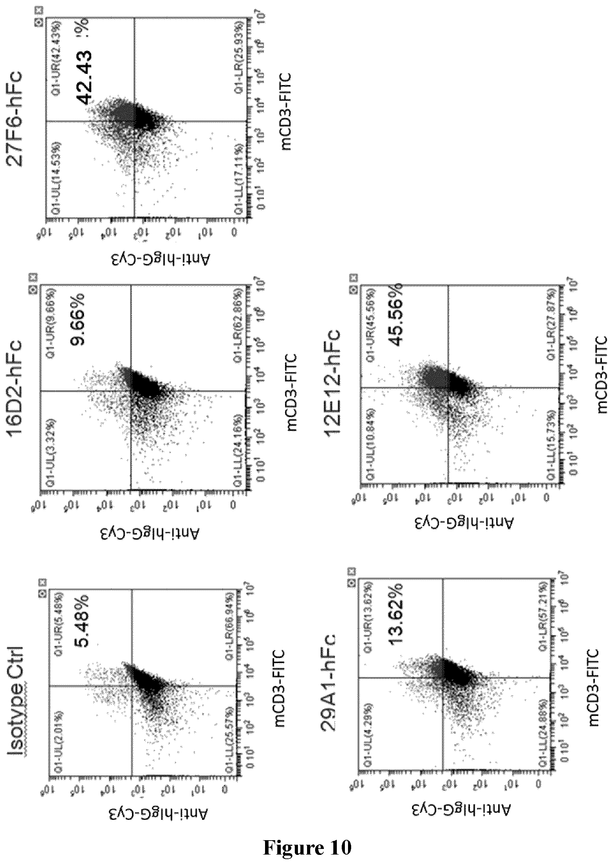

The experimental results are shown in Figure 10, and the percentage in the upper right quadrant represents the intensity of the binding of TNFR2 antibody to human T cell surface. The results showed that three chimeric TNFR2 monoclonal antibodies, 27F6-hFc, 29A1-hFc, and 12E12-hFc had strong binding ability to human TNFR2 on T cell surfaces, while 16D2-hFc had relatively weak binding ability. Iso (herceptin) is a specific antibody negative control. Compared to the negative control, the antibody of the present invention could specifically bind to the TNFR2 antigen on the surface of T cells.

### Example 6. Biofilm layer optical interference (BLI) technology study on the binding activity of human-mouse chimeric TNFR2 monoclonal antibody with human TNFR2.

The GATOR (ProbeLife) detection instrument and AHC (Pall, 185064), Human antibody Capture probe were used to detect the dissociation constant (Kd) of human-mouse chimeric TNFR2 monoclonal antibody.

The human-mouse chimeric TNFR2 monoclonal antibodies were diluted to 50nM in binding buffer (Q buffer [PBS (10mM PH7.4)+0.02% Tween 20+0.2% BSA). The recombinant human TNFR2 protein was diluted in Q buffer with a 2-fold gradient starting from the highest concentration of 100 nM. The kinetic association assay was conducted by placing the antibody capture sensor in the continuously diluted antigen solutions mentioned above. The Octet Data Acquisition software was pened and the New Kinetics Experiment-Basic Kinetics mode was selected.

**Table 3. Process for studying antibody binding activity using biofilm layer optical interferometry (BLI)**

| Process | Time | Concentration |
|---|---|---|
| Baseline 1 | 30 s | Q buffer |
| Loading | 120 s | 30 nM antibody (in the buffer) |
| Baseline 2 | 60 s | Q buffer |
| Association | 120 s | 2-fold dilution of antigen in Q buffer from 100 nM |
| Dissociation | 120 s | Q buffer |
| Regeneration | 25 s | Q buffer |

### Experimental results and analysis:

The results are shown in the table below:

**Table 4. Kinetic analysis results of human-mouse chimeric monoclonal antibody binding to human TNFR2**

| Loaded sample ID | K_{D} (M) | kon(1/Ms) | kdis(1/s) | Full R² | R_{Max} |
|---|---|---|---|---|---|
| 7F2-hFc | 2.53E-08 | 2.53E+05 | 6.39E-03 | 0.998 | 0.36 |
| 11G2-hFc | 3.04E-09 | 4.55E+05 | 1.38E-03 | 0.999 | 0.34 |
| 12E12-hFc | 3.90E-09 | 7.80E+05 | 3.04E-03 | 0.9966 | 0.361 |
| 27F6-hFc | 9.38E-09 | 8.11E+05 | 7.61E-03 | 0.984 | 0.255 |
| 29A1-hFc | 1.63E-08 | 2.89E+05 | 4.69E-03 | 0.9888 | 0.234 |
| 33C12-hFc | 2.19E-08 | 1.31E+05 | 2.86E-03 | 0.9954 | 0.207 |

The experimental results show that the correlation coefficient R² of all antibodies in the Global fitting mode is greater than 0.95, which meets the system adaptability requirements, and the results are reliable.

### Example 7. Human-mouse chimeric TNFR2 monoclonal antibody promotes Jurkat-TNFR2 secreting IL2

Jurkat cells are a type of T lymphocyte line, and they can secrete IL2 when activated. To test the activation function of the antibody of the present invention on T cells, we conducted the following tests.

Antibody coated in 96-well plate: the anti-CD3 mAb (Biogene, 317302) was diluted to 0.5µg/mL with PBS, and the antibodies of the present invention were diluted to 20, 2, 0.2, 0 µg/mL, respectively, then added to a 96-well plate at 40 µL/well, respectively, 4 °C overnight. The next day, the coating solution was sucked and discarded. The Jurkat-TNFR2 cells were counted, and added to the corresponding well at 2×10⁵ cells per well, 200 µL, and incubated at 37 °C with 5% CO₂ for 24 hours. After 24 hours, the supernatant was extracted and the content of IL2 was determined (Ekosai, catalog number EH002).

### Analysis of experimental results

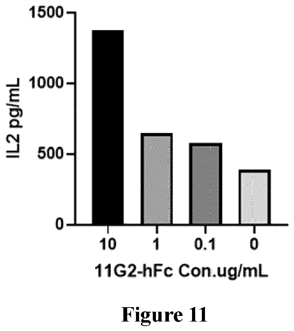

As shown in Figure 11, the antibody 11G2-hFc of the present invention could significantly promote the secretion of IL2 by Jurkat-TNFR2 cells. It proves that the antibody can promote the release of cytokines, enhance cell activity, and activate cells.

### Example 8: Human-mouse chimeric TNFR2 monoclonal antibody enhances IFNγ production of PBMC in vitro

After T cell activation, the cytokine IFNγ was secreted by lymphocytes. To test the function of the chimeric TNFR2 monoclonal antibody of the present invention as a positive regulator for T cell activation, the following experiment was conducted to demonstrate the effect the antibody of the present invention on enhancing the production of IFNγ in PBMC cells.

The commercially available frozen PBMC cells (Oricellls) were resuscitated, and the cell density was adjusted to 5×10⁶/mL. PHA was diluted to 2µg/mL using RPMI1640+10% FBS medium, the human-mouse chimeric TNFR2 antibodies were diluted by the above PHA working solution in gradient, added to the corresponding well of the 96-well plate at 100µL per well, and finally 100 µL of cell suspension was added to the corresponding well, gently mixed and incubated for 3 days in a 37 °C, 5% CO₂ incubator. After 3 days, 100µL supernatant was absorbed to detect IFNγ content (Ekosai, catalog number EH008).

### Experimental results and analysis

The results are shown in the table below:

**Table 5. Human-mouse chimeric TNFR2 monoclonal antibody enhances IFNγ production of PBMC in vitro**

| | Antibody conc. (µg/mL) | IFNγ (pg/mL) | Increased percentage |
|---|---|---|---|
| 27F6-hFc | 1 | 7497 | 47% |
| 33C12-hFc | 1 | 8965 | 76% |
| 11G2-hFc | 1 | 10110 | 98% |
| 12E12-hFc | 1 | 10828 | 113% |
| 7F2-hFc | 1 | 5608 | 10% |
| IgG1 | 1 | 5095 | 0% |
| Blank | -- | 4995 | -2% |

The results showed that human-mouse chimeric TNFR2 monoclonal antibodies 27F6, 33C12-hFc, 11G2-hFc, 12E12-hFc, and 7F2-hFc could all promote the secretion of IFNγ by T cells in PBMCs, and the increased percentage of IFNγ reached 10%-113%, compared to the negative control IgG1. The above results demonstrate that these antibodies can enhance the production of IFNγ of PBMC *in vitro,* promote the release of cytokines, enhance cell activity, and activate cells.

### Example 9. Effect of human-mouse chimeric TNFR2 monoclonal antibody on tumor cell activity

### 9.1 Effect of human-mouse chimeric TNFR2 monoclonal antibody on the activity of Jurkat-TNFR2 tumor cells

The human-mouse chimeric TNFR2 monoclonal antibody to be tested was diluted by complete culture medium (RPMI1640+10% FBS) and added to the corresponding well of 96-well plate. The dilution method for TNFα (Novoprotein, catalog number C008) is the same as above, with a starting concentration of 300ng/mL.

The Jurkat-TNFR2 cells with good growth status were counted, and the cell density was adjusted to 2×10⁶ cells/mL using fresh complete culture medium, then the cells were added to the corresponding 96 wells at 50µL cells per well. The above-mentioned cells were cultured at 37 °C with 5% CO₂, and the cell viability was measured after 48 hours. The detection reagent CellTiter-Glo Luminescent Cell Viability Assay (Promega, catalog number G7572) was used for testing.

A parallel experiment was conducted under the same conditions as above, to detect the number of live cells in Jurkat-TNFR2 cells treated with the method of the present invention at 24 and 48 hours. Cells was resuspended by PBS, then washed and stained with Annexin V and PI (BD, 51-6710AK), respectively. The number of live cells was detected by flow cytometry.

### Experimental results and analysis

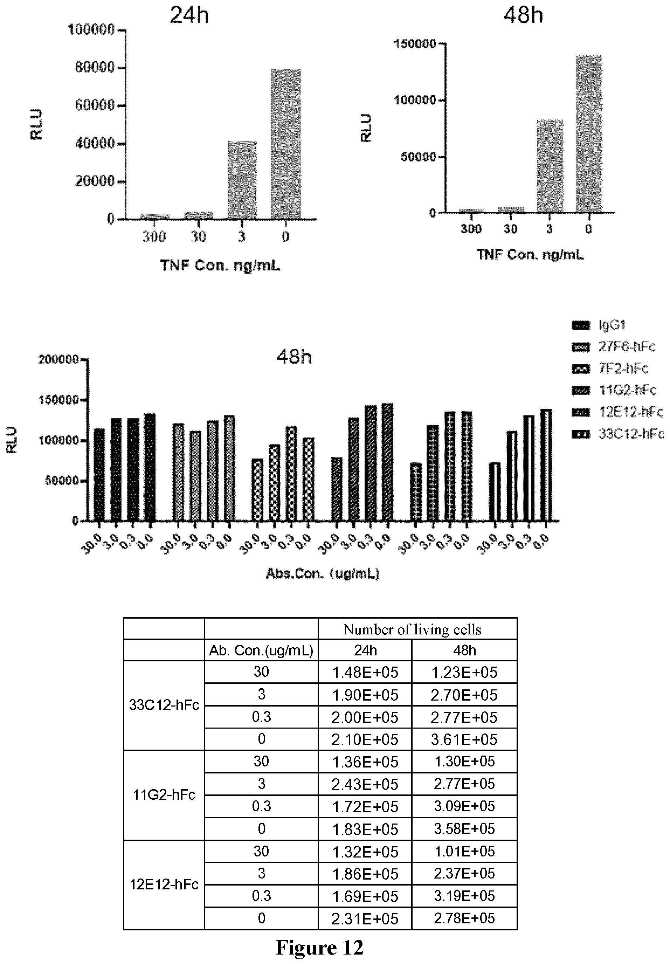

The results are shown in Figure 12, where TNFα shown in the upper figure is a positive control, which can induce apoptosis in Jurkat-TNFR2 tumor cells. The various monoclonal antibodies with different concentration gradients (from left to right: IgG1, 27F6-hFc, 7F2-hFc, 11G2-hFc, 12E12-hFc, 33C12-hFc) are shown in the figure below. Among them, 27F6-hFc had no effect on cell viability, while 7F2-hFc, 1102-hFc, 12E12-hFc, and 33C12-hFc had a significant effect on the cell viability of Jurkat-TNFR2. The numbers of live cells treated with different antibodies are shown in the table in the figure. Jurkat-TNFR2 cells treated with 11G2-hFc, 12E12-hFc, and 33C12-hFc showed a significant decrease in the number of live cells.

The above experiments indicate that the above antibodies can induce apoptosis of Jurkat-TNFR2 tumor cells and have an activity of killing tumor cells.

### 9.2 Effect of human-mouse chimeric TNFR2 monoclonal antibody on the activity of Jeko-1 tumor cells

In this experiment, Jeko-1 cells were used, which were human mantle cell lymphoma cells It has been reported that they express high levels of TNFR2 (Michael Yang et al., Optimizing TNFR2 antigen for immunotherapy with tumor microenvironment specificity, J Leukoc Biol. 2020; 1-10.).

Firstly, the TNFR2 expression level of Jeko-1 cells was validated: a certain number of Jeko-1 cells (purchased from the cell bank of Chinese Academy of Sciences) were washed, resuspended, and PE-anti-hTNFR2 antibody (Biogene, 358404) was added for incubation. Then, the cells were washed for flow cytometry detection.

Next, to investigate the effect of human-mouse chimeric TNFR2 monoclonal antibody on the activity of Jeko-1 cells: the human-mouse chimeric TNFR2 monoclonal antibodies to be tested were gradient diluted with complete culture medium (RPMI1640+10% FBS) and added to the corresponding well of 96-well plate. The Jeko-1 cells with good growth status were counted, and the cell density was adjusted to 2×10⁶ cells/mL using fresh complete culture medium, then the cells were added to the corresponding 96 wells at 100µL per well. The above cells were cultivated at 37 °C with 5% CO₂ for 3 days. After 3 days, cell viability was detected using CCK8 reagent (Beyotime, C0038).

### Experimental results and analysis

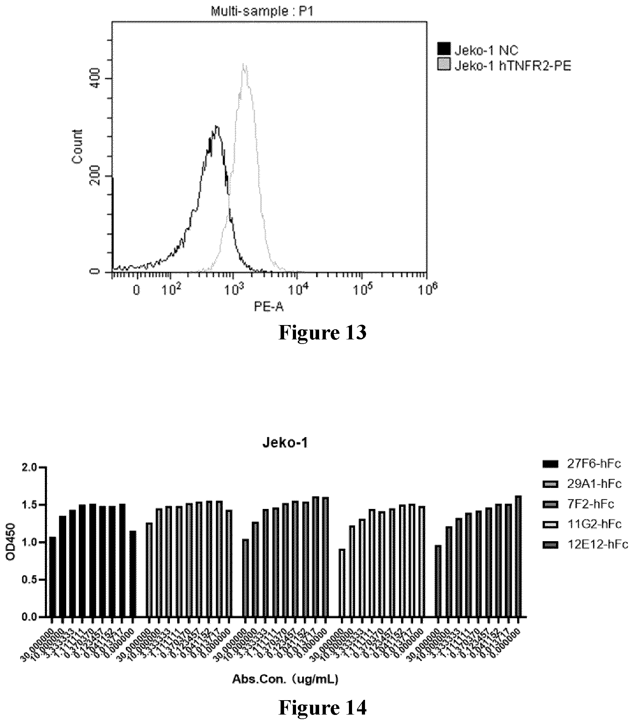

As shown in Figure 13, the Jeko-1 cells have high expression of TNFR2.

Figure 14 shows the effects of various monoclonal antibodies with different concentration gradients (from left to right: 27F6-hFc, 29A1-hFc, 7F2-hFc, 11G2-hFc, 12E12-hFc) on the activity of Jeko-1 cells. As shown in Figure 14, the human-mouse chimeric TNFR2 monoclonal antibodies 7F2-hFc, 11G2-hFc, and 12E12-hFc had a significant impact on the activity of Jeko-1 tumor cells, while 27F6-hFc and 29A1-hFc had little effect on the activity of Jeko-1 cells.

The above results indicate that the antibodies 7F2-hFc, 11 G2-hFc, and 12E12-hFc of the present invention can induce apoptosis of Jeko-1 tumor cells and have an activity of killing tumor cells.

### 9.3 The effect of the combination of TNFα and human-mouse TNFR2 monoclonal antibody on Jurkat-TNFR2 tumor cells

Next, the effect of the combination of TNFα and the human-mouse chimeric TNFR2 monoclonal antibody on the cell viability of Jurkat-TNFR2 tumor cells was detected. The fixed concentration of TNF was 3ng/mL, and the human-mouse chimeric TNFR2 monoclonal antibodies were gradient diluted. The method is the same as Example 9.1. IgG1 is an isotype negative control.

### Experimental results and analysis

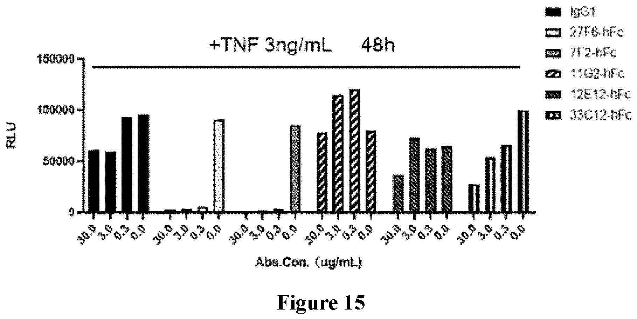

The experimental results are shown in Figure 15. The TNFR2 human-mouse chimeric monoclonal antibodies 27F6-hFc and 7F2-hFc could significantly promote the cell apoptosis caused by TNFα. The two had a strong synergistic effect on killing Jurkat tumor cells transfected with TNFR2, while other clones had no or weak synergistic effect.

### Example 10. Study on TNFR2 monoclonal antibody inhibiting tumor growth in mice in vivo

### 10.1 TNFR2 monoclonal antibody binding to T cells of human TNFR2 gene knock in (hTNFR2 KI) mice

The previous results showed that the monoclonal antibody of the present invention had no binding activity with TNFR2 in mice. To study the *in vivo* drug efficacy in mice, experiments were conducted using hTNFR2 knock in mice (hTNFR2 KI mice, purchased from BIOCYTOGEN Jiangsu Gene Biotechnology Co., Ltd.). Firstly, the binding activity of the monoclonal antibody of the present invention to T cells of hTNFR2 KI mice was detected.

The spleens of wild-type (WT) and hTNFR2 KI mice were taken, respectively, and the spleen cells were isolated, and incubated by ConA (Sigma, catalog number: C5275-5MG) with a final concentration of 2 µg/mL at 37 °C with 5% CO₂ for 48 hours. After 48 hours, the cells were collected for the following operation: Cy-3 anti-human IgG (Jakson 100-176-098) was added, and the final concentration of the antibody of the present invention was 10 µg/mL, and the volume was 100 µL, incubated at 4 °C for 30 minutes. The cells were stained again with Cy-3 anti-human IgG and FITC anti-mCD3 (BD, 555274) after washing, under the same incubation conditions. After washing, the detection was performed.

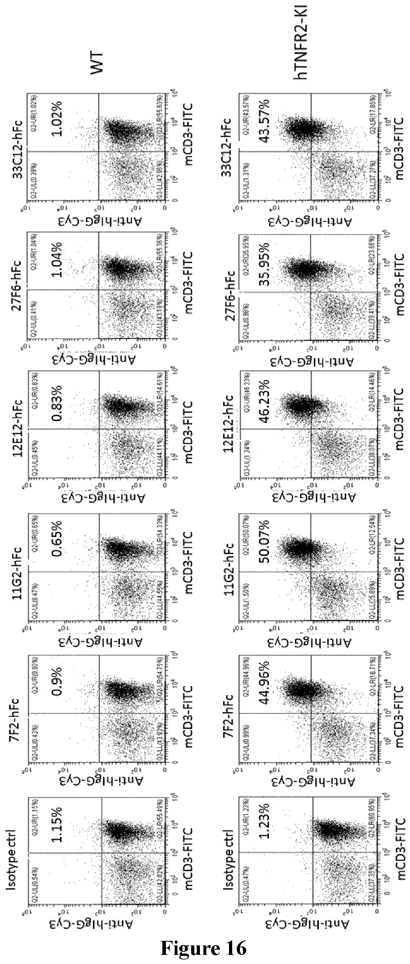

The results are shown in Figure 16. Isotype Ctrl did not bind to T cells of both WT and hTNFR2 KI mice, while the antibody of the present invention could specifically bind to T cells of hTNFR2 KI mice.

### 10.2 TNFR2 monoclonal antibody inhibits tumor growth in mice in vivo

Mouse colon cancer cells MC-38 (purchased from Shanghai Nanfang Model Biotechnology Co., Ltd.) were cultured in DMEM medium containing 10% FBS and passaged 2-3 times a week. On the day of mice inoculation, cells were treated with trypsin, centrifuged and precipitated, the supernatant was discarded, and the cells were washed, and finally suspended in PBS with a cell density of 1×10⁷ cells/ml.

Then, MC-38 cells were subcutaneously transplanted into mice, and 0.1ml of cell suspension containing 1×10⁶ cells was injected subcutaneously into the back of each mouse. The administration method for mice was intraperitoneal injection, with a volume of 0.2ml (TNFR2 monoclonal antibody injection, 1mg/ml) each time. On the 6th day after inoculation, the mice were randomly grouped according to tumor volume, and given tail vein injection twice a week for a total of 4 doses. The weight and tumor volume of mice were measured twice a week (during the administration period, the weight and tumor volume were measured before administration). The detailed administration information of each group of experimental animals was given in the table below.

**Table 6. Administration information of experimental animals in each group**

| **Group** | | **Dosage µg/dose** | | **Number of mice** | **Administration time point** |
|---|---|---|---|---|---|
| Number | Category | Dosage µg/dose | Dosage volume | | |
| 1 | PBS | | 200 µl/mouse | 8 mice/group, half male and half female | When the tumor volume of each group of mice was about 100mm³, the mice were randomly grouped according to the tumor volume, and administered twice a week with an interval of 2-3 days. 4 times in total, administered by intraperitoneal and tail vein injection |
| 2 | 13F5-hFc | 200 | | | |
| 3 | 33C12-hFc | 200 | | | |
| 4 | 7F2-hFc | 200 | | | |
| 5 | 11G2-hFc | 200 | | | |
| 6 | 12E12-hFc | 200 | | | |
| 7 | 27F6-hFc | 200 | | | |
| 8 | IgG1 | 200 | | | |

The body weight data, tumor volume, and tumor weight data of the experimental and control groups of mice were presented in the form of arithmetic mean ± standard deviation). T-test analysis was performed on inter group data to determine if there were significant differences between the groups.

### Experimental results and analysis

In this experiment, the body weight data of mice were analyzed for inter group statistical differences at each time point after administration. The P-values of the TNFR2 monoclonal antibody dose group, PBS group, and IgG1 group were all greater than 0.05, indicating that the administration had no effect on the body weight of mice. On day 24, all mice were euthanized and their tumors and spleens were dissected and weighed. The tumor weight values of each group of mice are presented in the form of arithmetic mean ± standard deviation; T-test was used to analyze the statistical differences between the experimental group and the control group, with a tumor weight difference of TEST-P<0.05 in the 33C12 group.

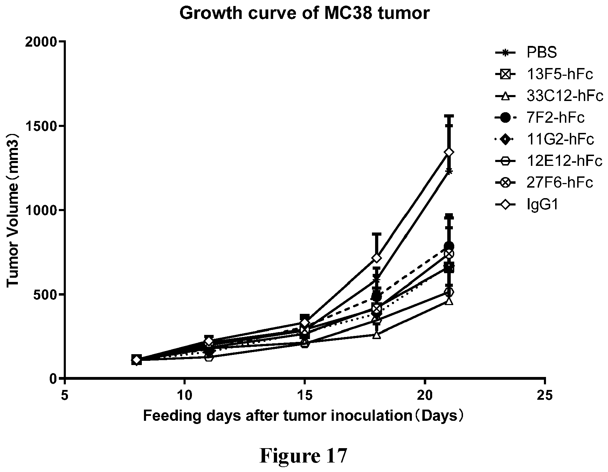

Figure 17 shows the line graph of tumor volume over time in mice treated with TNFR2 mouse monoclonal antibody. As shown in Figure 17, after 4 doses of administration, 6 strains of TNFR2 monoclonal antibodies (13F5, 33C12, 7F2, 11G2, 12E12, 27F6) showed inhibitory effects on MC-38 tumors compared to PBS group and IgG1 group. Through the analysis of tumor volume, the relative tumor inhibition rates of the 33C12 and 12E12 groups reached about 40%, the relative tumor inhibition rates of the 11G2 and 13F5 groups reached about 55%, and the 27F6 and 7F2 groups were slightly worse. One week after the end of administration, the relative tumor inhibition rate of 33C12 was 48%, indicating that the 33C12 clone has a significant inhibitory effect on MC-38 tumors. Statistical difference analysis was conducted on the tumor volume data of mice at different time points, and there was a significant statistical difference in the TEST analysis value between the 33C12 group and the PBS group, with a P value of less than 0.05 (P=0.018813). 33C12 showed a more significant difference compared to IgG1 groups, with P ≤ 0.02.

### Example 11. Design and preparation of humanized anti-human TNFR2 monoclonal antibody

### 11.1 Design of humanized anti-human TNFR2 monoclonal antibody

Based on the maternal mouse antibody sequences of 11G2, 33C12, and 12E12, humanized antibody sequences were designed. The specific steps are as follows: firstly, a three-dimensional molecular model of the variable region was constructed by homologous modeling methods using Discovery Studio and Schrödinger Antibody Modeling. Next, by comparing the existing antibody structures in the database, the structure of the maternal antibody variable region and CDR was simulated. Meanwhile, Germline sequences derived from cDNA with high homology to mouse maternal antibody VH and VL were selected for alignment. The IGHV1 with the highest homology was selected as the humanized design template of the heavy chain VH of 11G2, and the sequence was designed. The IGKV1 was selected as the humanized design template for light chain VL, and the sequence was designed. The IGHV3 with the highest homology was selected as the humanized design template for heavy chain VH of 33C12, and sequence was designed. The IGKV3 was selected as the humanized design template for the light chain VL, and the sequence was designed. The IGHV1 with the highest homology was selected as the humanized design template for the heavy chain VH of 12E12, and the sequence was designed. The IGLV7 and IGKV3 were selected as humanized design templates for the light chain VL, and the sequences were designed.

### Results and Discussion

Five humanized sequences were designed based on the original heavy chain mVH sequence of mouse maternal antibody number **11G2:** 11G2-huVH1 (SEQ ID NO: 73), 11G2-huVH12 (SEQ ID NO: 74), 11G2-huVH3 (SEQ ID NO: 75), 11 G2-huVH4 (SEQ ID NO: 76), and 11 G2-huVH5 (SEQ ID NO: 77), wherein SEQ ID NOs: 73-77 had the sequence identity of 75.6%-85.7% with the original mVH sequence SEQ ID NO: 33. Three humanized sequences were designed based on the original light chain mVL of the antibody: 11G2-huVL1 (SEQ ID NO: 78), 11 G2-huVL2 (SEQ ID NO: 79), and 11 G2-huVL3 (SEQ ID NO: 80), wherein the sequence identity between SEQ ID NO: 78-80 and the original mVL was 76.6-83.2%.

**Table 7. VH and VL sequences of humanized antibody based on 11G2**

| **Description** | | **SEQ ID NO** | **Sequence** |
|---|---|---|---|
| 11G2-huVH1 | Full length | 73 | QVQLVQSGAEVKKPGASVKLSCKASGYTFITYDINWVR QAPGQGLEWIGWIYPRDGNTQFNENFKGKATMTVDES TSTAYMEVRSLRSDDTAVYYCARGDRDYGYFDVWGQG TTVTVSS |
| | CDR1 | 34 | TYDIN |
| | CDR2 | 35 | WIYPRDGNTQFNENFKG |
| | CDR3 | 36 | GDRDYGYFDV |
| 11G2-huVH2 | Full length | 74 | QVQLVQSGAEVKKPGASVKVSCKASGYTFITYDINWVR QAPGQGLEWIGWIYPRDGNTQFNENFKGRVTMTTDTST STAYMELRSLRSDDTAVYYCARGDRDYGYFDVWGQG TTVTVSS |
| | CDR1 | 34 | TYDIN |
| | CDR2 | 35 | WIYPRDGNTQFNENFKG |
| | CDR3 | 36 | GDRDYGYFDV |
| 11G2-huVH3 | Full length | 75 | QVQLVQSGAEVKKPGASVKVSCKASGYTFITYDINWVR QATGQGLEWIGWIYPRDGNTQFNENFQGRVTMTRNTSI STAYMELSSLRSEDTAVYYCARGDRDYGYFDVWGQGT TVTVSS |
| | CDR1 | 34 | TYDIN |
| | CDR2 | 87 | WIYPRDGNTQFNENFQGR |
| | CDR3 | 36 | GDRDYGYFDV |
| 11G2-huVH4 | Full length | 76 | QVQLVQSGAEVKKPGASVKVSCKASGYTFITYDINWVR QAPGQGLEWIGWIYPRDGNTQYAQKFQGRVTMTTDTS TSTAYMELRSLRSDDTAVYYCARGDRDYGYFDVWGQ GTTVTVSS |
| | CDR1 | 34 | TYDIN |
| | CDR2 | 88 | WIYPRDGNTQYAQKFQGR |
| | CDR3 | 36 | GDRDYGYFDV |
| 11G2-huVH5 | Full length | 77 | QVQLVQSGAEVKKPGASVKVSCKASGYTFITYDINWVR QATGQGLEWIGWIYPRDGNTQYAQKFQGRVTMTRNTS ISTAYMELSSLRSEDTAVYYCARGDRDYGYFDVWGQG TTVTVSS |
| | CDR1 | 34 | TYDIN |
| | CDR2 | 88 | WIYPRDGNTQYAQKFQGR |
| | CDR3 | 36 | GDRDYGYFDV |
| 11G2-huVL1 | Full length | 78 | DIQMTPSTSSLSASVGDRVTITCRASQDISNYLNWY QQKPGKAVKLLIYFKSKLHSGVPSRFSGSGSGTDFT LTISSLQPEDFATYYCQQGNTLPLTFGGGTKLELK |
| | CDR1 | 38 | RASQDISNYLN |
| | CDR2 | 39 | FKSKLHS |
| | CDR3 | 40 | QQGNTLPLT |
| 11G2-huVL2 | Full length | 79 | DIQMTQSPSSLSASVGDRVTITCRASQDISNYLNWY QQKPGKAPKLLIYFKSKLHSGVPSRFSGSGSGTDFT LTISSLQPEDFATYYCQQGNTLPLTFGGGTKLEIK |
| | CDR1 | 38 | RASQDISNYLN |
| | CDR2 | 39 | FKSKLHS |
| | CDR3 | 40 | QQGNTLPLT |
| 11G2-huVL3 | Full length | 80 | DIQMTQSPSSLSASVGDRVTITCQASQDISNYLNWY QQKPGKAPKLLIYFKSKLETGVPSRFSGSGSGTDFT FTISSLQPEDIATYYCQQGNTLPLTFGGGTKLEIK |
| | CDR1 | 38 | QASQDISNYLN |
| | CDR2 | 89 | FKSKLET |
| | CDR3 | 40 | QQGNTLPLT |

Three humanized sequences were designed based on the original heavy chain mVH sequence of mouse maternal antibody **33C12:** 33c12-huVH1 (SEQ ID NO: 81), 33c12-huVH2 (SEQ ID NO: 82), and 33c12-huVH3 (SEQ ID NO: 83), wherein the sequence identity between SEQ ID NOs: 81-83 and the original mVH sequence SEQ ID NO: 65 was 89.1-92.5%. Three humanized sequences were designed based on the original light chain mVL: 33c12-huVL1 (SEQ ID NO: 84), 33c12-huVL1 (SEQ ID NO: 85), and 33c12-huVL3 (SEQ ID NO: 86), wherein the sequence identity between SEQ ID NOs: 84-86 and the original mVH sequence SEQ ID NO: 66 was 75.0%-82.4%.

**Table 8. VH and VL sequences of humanized antibody based on 33C12**

| **Description** | | **SEQ ID NO** | **Sequence** |
|---|---|---|---|
| 33C12-huVH1 | Full length | 81 | DVQLVESGGGLVKPGGSLRLSCAASGFTFSDYTMSWV RQAPGKGLEWVATISTGGSYTYYPDSVKGRFTISRDNA KNTLYLQMSSLRAEDTAVYYCTRDGGYDGDYFDYW GQGTTVTVSS |
| | CDR1 | 66 | DYTMS |
| | CDR2 | 67 | TISTGGSYTYYPDSVKG |
| | CDR3 | 68 | DGGYDGDYFDY |
| 33C12-huVH2 | Full length | 82 | EVQLVESGGGLVKPGGSLRLSCAASGFTFSDYTMSWV RQAPGKGLEWVATISTGGSYTYYPDSVKGRFTISRDNA KNTLYLQMSSLRAEDTAVYYCARDGGYDGDYFDYW GQGTTVTVSS |
| | CDR1 | 66 | DYTMS |
| | CDR2 | 67 | TISTGGSYTYYPDSVKG |
| | CDR3 | 68 | DGGYDGDYFDY |
| 33C12-huVH3 | Full length | 83 | EVQLVESGGGLVKPGGSLRLSCAASGFTFSDYTMSWV RQAPGKGLEWVATISTGGSYTYYADSVKGRFTISRDN AKNSLYLQMNSLRAEDTAVYYCARDGGYDGDYFDY WGQGTTVTVSS |
| | CDR1 | 66 | DYTMS |
| | CDR2 | 90 | TISTGGSYTYYADSVKG |
| | CDR3 | 68 | DGGYDGDYFDY |
| 33C12-huVL1 | Full length | 84 | ENVLTQSPATLSLSPGERATMSCRASSSVSSSYLHWYQ QKPGQAPRLWIYRTSNLASGVPARFSGSGSGTDYTLTI SRVEPEDFAVYYCQQYSGYPLTFGGGTKLEIK |
| | CDR1 | 70 | RASSSVSSSYLH |
| | CDR2 | 71 | RTSNLAS |
| | CDR3 | 72 | QQYSGYPLT |
| 33C12-huVL2 | Full length | 85 | ENVLTQSPATLSLSPGERATLSCRASSSVSSSYLHWYQ QKPGQAPRLWIYRTSNLASGVPARFSGSGSGTDFTLTIS RLEPEDFAVYYCQQYSGYPLTFGGGTKLEIK |
| | CDR1 | 70 | RASSSVSSSYLH |
| | CDR2 | 71 | RTSNLAS |
| | CDR3 | 72 | QQYSGYPLT |
| 33C12-huVL3 | Full length | 86 | EIVLTQSPATLSLSPGERATLSCRASSSVSSSYLHWYQQ KPGQAPRLLIYRTSNLATGIPDRFSGSGSGTDFTLTISRL EPEDFAVYYCQQYSGYPLTFGGGTKLEIK |
| | CDR1 | 70 | RASSSVSSSYLH |
| | CDR2 | 91 | RTSNLAT |
| | CDR3 | 72 | QQYSGYPLT |

Eight humanized sequences were designed based on the original heavy chain mVH sequence of mouse maternal antibody 12E12: 112E12-huVH1 (SEQ ID NO: 92), 12E12-huVH2 (SEQ ID NO: 93), 12E12-huVH3 (SEQ ID NO: 94), 12E12-huVH4 (SEQ ID NO: 96), 12E12-huVH5 (SEQ ID NO: 97), 12E12-huVH6 (SEQ ID NO: 99), 12E12-huVH7 (SEQ ID NO: 100), 12E12-huVH8 (SEQ ID NO: 101); Five humanized sequences were designed based on the original light chain mVL: 112E12-huVL1 (SEQ ID NO: 102), 12E12-huVL2 (SEQ ID NO: 103), 12E12-huVL3 (SEQ ID NO: 104), 12E12-huVL4 (SEQ ID NO: 105), 12E12-huVL5 (SEQ ID NO: 106).

**Table 9: VH and VL sequences of humanized antibody based on 12E12**

| **Description** | | **SEQ ID NO** | **Sequence** |
|---|---|---|---|
| 12E12-huVH1 | Full length | 92 | EVQLVQSGAEVKKPGASVKLSCTVSGFNIKDFYIHWVRQA PGQGLEWIGRVDPEDGNTEYAPNFQDRATMTTDTSTNTAYL ELSSLRSEDTAVYYCSTWDGFSYFDYWGQGTTVTVSS |
| | CDR1 | 42 | DFYIH |
| | CDR2 | 43 | RVDPEDGNTEYAPNFQD |
| | CDR3 | 44 | WDGFSYFDY |
| 12E12-huVH2 | Full length | 93 | QVQLVQSGAEVKKPGASVKVSCKVSGFNIKDFYIHWVRQA PGQGLEWIGRVDPEDGNTEYAPNFQDRATMTTDTSTSTAYL ELSSLRSEDTAVYYCSTWDGFSYFDYWGQGTTVTVSS |
| | CDR1 | 42 | DFYIH |
| | CDR2 | 43 | RVDPEDGNTEYAPNFQD |
| | CDR3 | 44 | WDGFSYFDY |
| 12E12-huVH3 | Full length | 94 | QVQLVQSGAEVKKPGASVKVSCKVSGFNIKDFYIHWVRQA PGKGLEWMGRVDPEDGNTEYAQKFQGRVTMTTDTSTDTA YMELSSLRSEDTAVYYCSTWDGFSYFDYWGQGTTVTVSS |
| | CDR1 | 42 | DFYIH |
| | CDR2 | 95 | RVDPEDGNTEYAQKFQG |
| | CDR3 | 44 | WDGFSYFDY |
| 12E12-huVH4 | Full length | 96 | EVQLVQSGAEVKKPGATVKISCKVSGFNIKDFYIHWVQQAP GKGLEWIGRVDPEDGNTEYAPNFQDRVTITADTSTDTAYME LSSLRSEDTAVYYCSTWDGFSYFDYWGQGTTVTVSS |
| | CDR1 | 42 | DFYIH |
| | CDR2 | 43 | RVDPEDGNTEYAPNFQD |
| | CDR3 | 44 | WDGFSYFDY |
| 12E12-huVH5 | Full length | 97 | EVQLVQSGAEVKKPGATVKISCKVSGFNIKDFYIHWVQQAP GKGLEWIGRVDPEDGNTEYAEKFQGRVTITADTSTDTAYME LSSLRSEDTAVYYCSTWDGFSYFDYWGQGTTVTVSS |
| | CDR1 | 42 | DFYIH |
| | CDR2 | 98 | RVDPEDGNTEYAEKFQG |
| | CDR3 | 44 | WDGFSYFDY |
| 12E12-huVH6 | Full length | 99 | QVQLVQSGAEVKKPGASVKVSCKASGYTITDFYIHWVRQA PGQGLEWIGRVDPEDGNTEYAPNFQDRATMTTDTSTSTAYL ELSSLRSEDTAVYYCSTWDGFSYFDYWGQGTTVTVSS |
| | CDR1 | 42 | DFYIH |
| | CDR2 | 43 | RVDPEDGNTEYAPNFQD |
| | CDR3 | 44 | WDGFSYFDY |
| 12E12-huVH7 | Full length | 100 | QVQLVQSGAEVKKPGASVKVSCKASGYTITDFYIHWVRQA PGKGLEWMGRVDPEDGNTEYAQKFQGRVTMTTDTSTDTA YMELSSLRSEDTAVYYCSTWDGFSYFDYWGQGTTVTVSS |
| | CDR1 | 42 | DFYIH |
| | CDR2 | 95 | RVDPEDGNTEYAQKFQG |
| | CDR3 | 44 | WDGFSYFDY |
| 12E12-huVH8 | Full length | 101 | EVQLVQSGAEVKKPGATVKISCKVSGYTITDFYIHWVQQAP GKGLEWMGRVDPEDGNTEYAPNFQDRVTITADTSTDTAYM ELSSLRSEDTAVYYCSTWDGFSYFDYWGQGTTVTVSS |
| | CDR1 | 42 | DFYIH |
| | CDR2 | 43 | RVDPEDGNTEYAPNFQD |
| | CDR3 | 44 | WDGFSYFDY |
| 12E12-huVL1 | Full length | 102 | QAVVTQEPSLTVSPGGTVTLTCRSSTGPVTTNNYANWVQQK PGQAFRGLIGGTNDRAPGVPARFSGSLIGDKTALTLSGVQPE DEAEYYCALWYRNHWVFGGGTKLTVL |
| | CDR1 | 46 | RSSTGPVTTNNYAN |
| | CDR2 | 47 | GTNDRAP |
| | CDR3 | 48 | ALWYRNHWV |
| 12E12-huVL2 | Full length | 103 | QAVVTQEPSLTVSPGGTVTLTCRSSTGPVTTNNYANWVQQK PGQAFRGLIGGTNDRAPGVPARFSGSLLGGKAALTLSGAQP EDEAEYYCALWYRNHWVFGGGTKLTVL |
| | CDR1 | 46 | RSSTGPVTTNNYAN |
| | CDR2 | 47 | GTNDRAP |
| | CDR3 | 48 | ALWYRNHWV |
| 12E12-huVL3 | Full length | 104 | QAVVTQEPSLTVSPGGTVTLTCRSSTGPVTTNNYANWFQQK PGQAPRTLIGGTNDRAPGVPARFSGSLLGGKAALTLSGAQP EDEAEYYCALWYRNHWVFGGGTKLTVL |
| | CDR1 | 46 | RSSTGPVTTNNYAN |
| | CDR2 | 47 | GTNDRAP |
| | CDR3 | 48 | ALWYRNHWV |
| 12E12-huVL4 | Full length | 105 | EAVVTQSPATLSLSPGERATLSCRSSTGPVTTNNYANWVQQ KPGQAFRGLIGGTNDRAPGIPARFSGSGSGTDTTLTISSLQPE DFAVYYCALWYRNHWVFGGGTKLEIK |
| | CDR1 | 46 | RSSTGPVTTNNYAN |
| | CDR2 | 47 | GTNDRAP |
| | CDR3 | 48 | ALWYRNHWV |
| 12E12-huVL5 | Full length | 106 | EIVMTQSPATLSLSPGERATLSCRSSTGPVTTNNYANWYQQ KPGQAPRLLIGGTNDRATGIPARFSGSGSGTDTTLTISSLQPE DFAVYYCALWYRNHWVFGGGTKLEIK |
| | CDR1 | 46 | RSSTGPVTTNNYAN |
| | CDR2 | 107 | GTNDRAT |
| | CDR3 | 48 | ALWYRNHWV |

| | | | |
|---|---|---|---|
| Note: The underline indicates the CDR part, and the gray shadow indicates the CDR sequence that has changed compared to the original CDR during the humanization process. | | | |

The humanized VH and VL mentioned above were combined to obtain the corresponding humanized antibody:

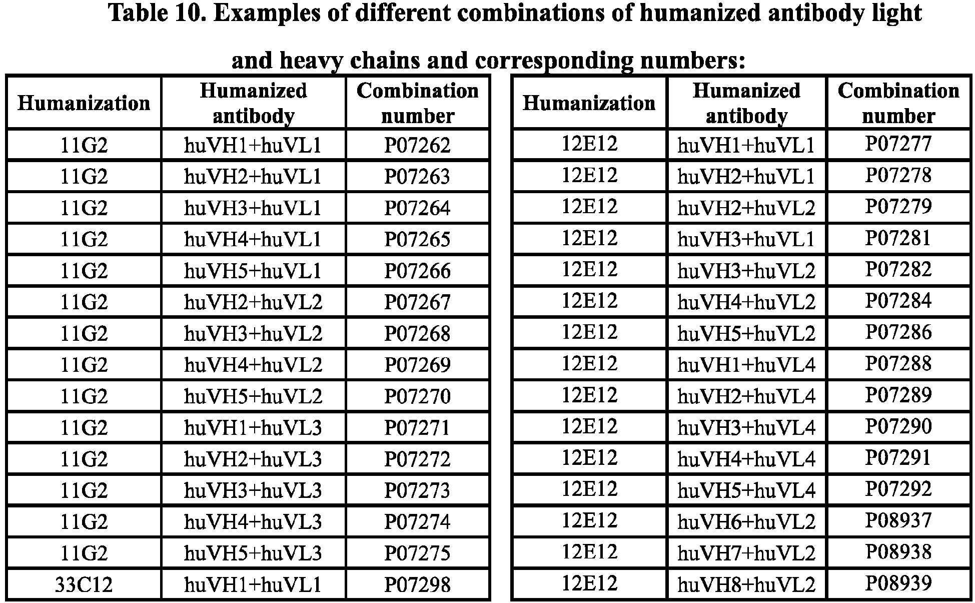

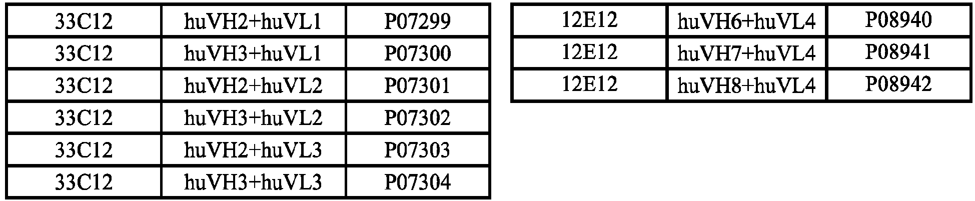

### 11.2 Construction of expression vector

Based on the humanized design results mentioned above, the sequences were constructed into the pcDNA3.4 vector, and the plasmid was obtained by PCR, enzyme digestion, ligation, transformation, identification, sequencing, alignment, and extraction.

### 11.3 Preparation of various combinations of antibodies

According to the humanized expression combinations shown in Table 10, the above plasmids were expressed in ExpiCHO-S cells for 7 days using the ExpiCHO-S expression system (Thermo Fisher, A29133) as required. On the 6th day of expression, the expression level was measured by HPLC. Finally, the antibody protein to be tested was purified through Protein A affinity chromatography in one step.

3 µg antibody to be tested was added to 5 x SDS loading buffer and 5 x SDS loading buffer with DTT of a final concentration of 50mM, heated at 100 °C for 3 minutes, and then electrophoresis was performed with 4%-12% SDS-PAGE gel. After stained by Coomassie brilliant blue, the gel was decolorized for imaging.

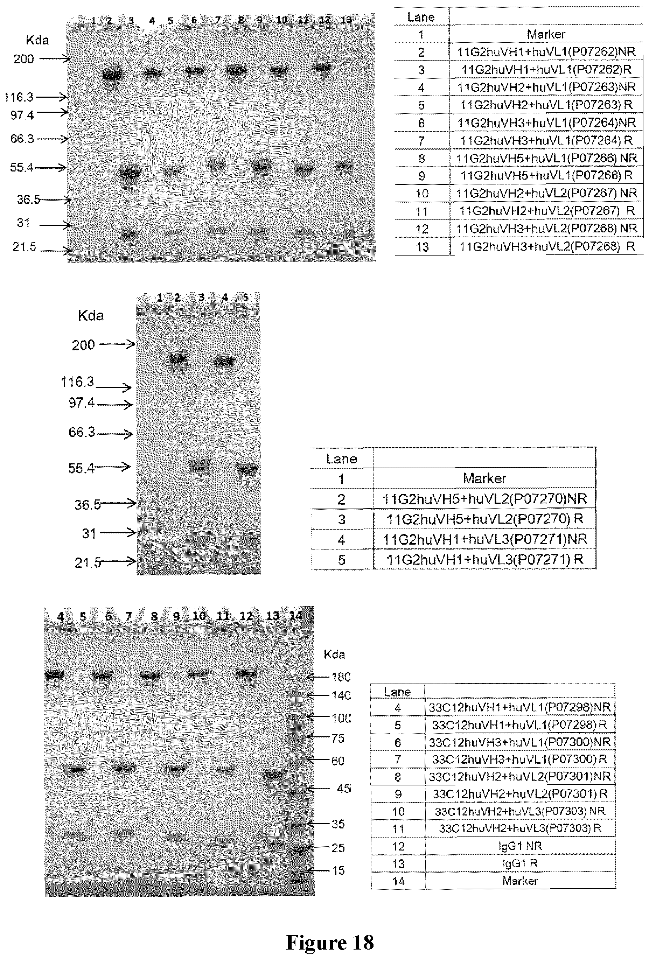

As shown in Figure 18, both the SDS-PAGE bands expressing Non-reducing and Reducing humanized antibodies conform to typical characteristics of monoclonal antibody,

### Example 12: Binding of humanized anti-human TNFR2 monoclonal antibody to recombinant human TNFR2

### 12.1 ELISA study on the in vitro binding activity of humanized anti-human TNFR2 monoclonal antibody with recombinant human TNFR2

To determine whether the humanized TNFR2 monoclonal antibody of the present disclosure can bind to recombinant human TIGIT protein, *in vitro* testing was conducted. The human-mouse chimeric antibody was used as a positive control. The experimental method is the same as Example 3.1

### Experimental results and analysis

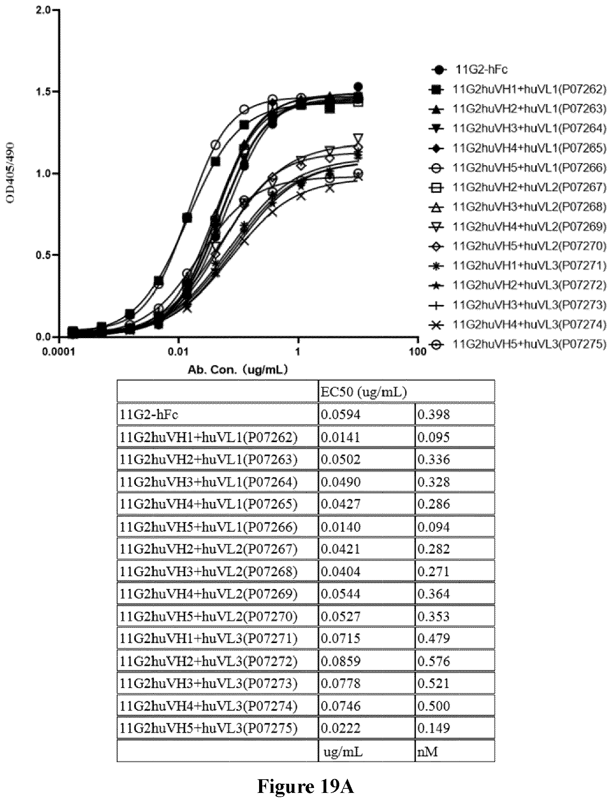

The ELISA test results of 14 strains of 11G2 anti-human TNFR2 humanized monoclonal antibodies (number P07262-P07275) are shown in Figure 19A. These humanized monoclonal antibodies could specifically bind to human TNFR2 with binding affinity EC₅₀ of 0.095 nM, 0.336 nM, 0.328 nM, 0.286 nM, 0.094 nM, 0.282 nM, 0.271 nM, 0.364 nM, 0.353 nM, 0.479 nM, 0.576 nM, 0.521 nM, 0.500 nM, and 0.149 nM, respectively.

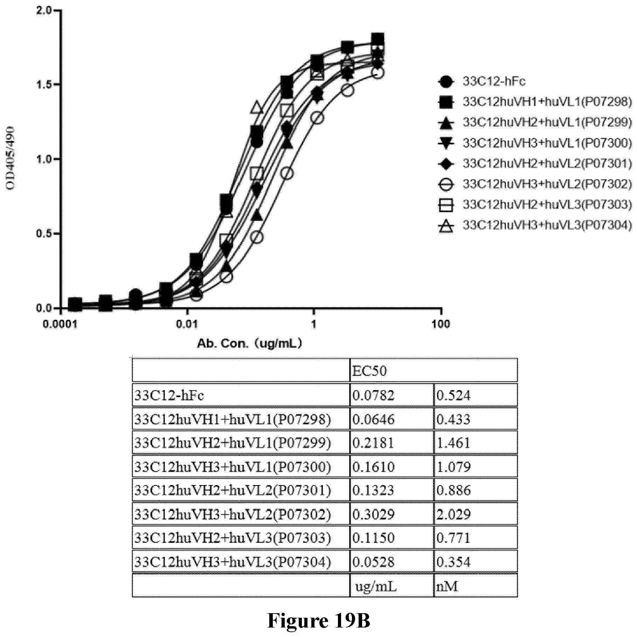

The ELISA test results of 7 strains of 33C12 anti-human TNFR2 humanized monoclonal antibodies (number P07298-P07304) are shown in Figure 19B. These humanized monoclonal antibodies could specifically bind to human TNFR2 with binding affinity EC₅₀ of 0.433 nM, 1.461 nM, 1.079 nM, 0.886 nM, 2.029 nM, 0.771 nM, and 0.354 nM, respectively.

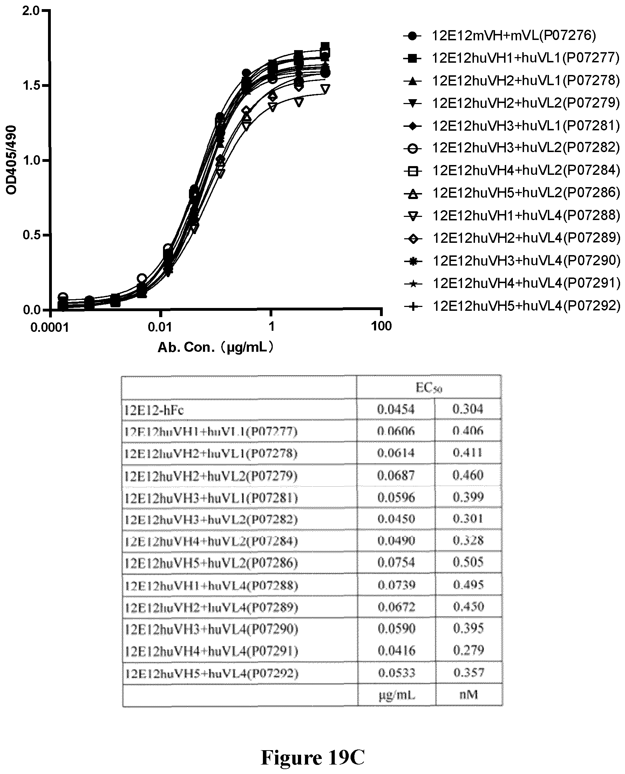

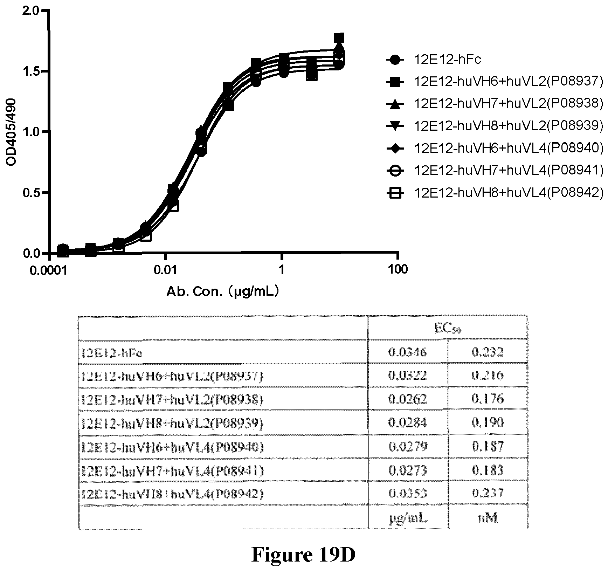

As for 12E12 anti-human TNFR2 humanized monoclonal antibodies, two independent binding tests were performed. In the first test, the ELISA test results of 12 strains of 12E12 anti-human TNFR2 humanized monoclonal antibodies are shown in Figure 19C. These humanized monoclonal antibodies could specifically bind to human TNFR2 with binding affinity EC₅₀ of 0.406 nM, 0.411 nM, 0.460 nM, 0.399 nM, 0.301 nM, 0.328 nM, 0.505 nM, 0.495 nM, 0.450 nM, 0.395 nM, 0.279 nM, and 0.357 nM, respectively. In the second test, the ELISA test results of 6 strains of 12E12 anti-human TNFR2 humanized monoclonal antibodies are shown in Figure 19D. These humanized monoclonal antibodies could specifically bind to human TNFR2 with binding affinity EC₅₀ of 0.216 nM, 0.176 nM, 0.190 nM, 0.187 nM, 0.183 nM, and 0.237 nM, respectively.

The above results indicate that the recombinant humanized antibody can bind to the antigen TNFR2, and the binding activity is equivalent to or better than that of the original human-mouse chimeric antibody.

### 12.2 Biofilm layer optical interference (BLI) technology study on the in vitro binding activity of humanized anti-human TNFR2 monoclonal antibody with TNFR2.

The experimental method is the same as Example 6.

The kinetic analysis results of humanized monoclonal antibodies binding to human TNFR2 are shown in the table below:

**Table 11. Kinetic analysis results of humanized antibodies designed based on maternal mouse antibody 11G2**

| **Sample number** | **TNFR2-His** | | | | |
|---|---|---|---|---|---|
| | **KD (M)** | **ka(1/Ms)** | **kd(1/s)** | **R²** | **Rₘₐₓ (nm)** |
| 11G2-hFc | 2.99E-09 | 3.72E+05 | 1.11E-03 | 0.998 | 0.889 |
| 11G2huVH1+huVL1(P07262) | 4.79E-09 | 2.39E+05 | 1.15E-03 | 0.999 | 1.25 |
| 11G2huVH2+huVL1(P07263) | 5.96E-09 | 2.87E+05 | 1.71E-03 | 0.999 | 0.986 |
| 11G2huVH3+huVL1(P07264) | 5.10E-09 | 3.16E+05 | 1.61E-03 | 0.999 | 0.88 |
| 11G2huVH4+huVL1(P07265) | 9.76E-09 | 2.61E+05 | 2.55E-03 | 0.998 | 0.958 |
| 11G2huVH5+huVL1(P07266) | 7.29E-09 | 2.23E+05 | 1.63E-03 | 0.999 | 1.1 |
| 11G2huVH2+huVL2(P07267) | 7.30E-09 | 2.80E+05 | 2.05E-03 | 0.998 | 0.968 |
| 11G2huVH3+huVL2(P07268) | 7.28E-09 | 3.02E+05 | 2.20E-03 | 0.997 | 0.871 |
| 11G2huVH4+huVL2(P07269) | 1.35E-08 | 2.81E+05 | 3.79E-03 | 0.996 | 0.89 |
| 11G2huVH5+huVL2(P07270) | 8.13E-09 | 3.26E+05 | 2.65E-03 | 0.997 | 0.784 |
| 11G2huVH1+huVL3(P07271) | 8.40E-09 | 2.99E+05 | 2.51E-03 | 0.998 | 0.868 |
| 11G2huVH2+huVL3(P07272) | 1.06E-08 | 2.76E+05 | 2.93E-03 | 0.997 | 0.883 |
| 11G2huVH3+huVL3(P07273) | 1.02E-08 | 2.49E+05 | 2.55E-03 | 0.998 | 0.899 |
| 11G2huVH4+huVL3(P07274) | 2.01E-08 | 2.54E+05 | 5.11E-03 | 0.995 | 0.827 |
| 11G2huVH5+huVL3(P07275) | 1.41E-08 | 2.13E+05 | 3.02E-03 | 0.998 | 1 |

**Table 12. Kinetic analysis results of humanized antibodies designed based on maternal mouse antibody 33C12**

| **Sample number** | **TNFR2-His** | | | | |
|---|---|---|---|---|---|
| | **KD (M)** | **ka(1/Ms)** | **kd(1/s)** | **R²** | **Rₘₐₓ (nm)** |
| 33C12-hFc | 1.73E-08 | 9.05E+04 | 1.56E-03 | 0.998 | 0.706 |
| 33C12huVH1+huVL1(P07298) | 1.77E-08 | 9.64E+04 | 1.71E-03 | 0.999 | 0.56 |
| 33C12huVH2+huVL1(P07299) | 1.51E-08 | 8.61E+04 | 1.30E-03 | 0.998 | 0.4 |
| 33C12huVH3+huVL1(P07300) | 1.27E-08 | 1.75E+05 | 2.23E-03 | 0.998 | 0.606 |
| 33C12huVH2+huVL2(P07301) | 1.78E-08 | 1.57E+05 | 2.79E-03 | 0.997 | 0.516 |
| 33C12huVH3+huVL2(P07302) | 1.19E-08 | 1.58E+05 | 1.88E-03 | 0.995 | 0.466 |
| 33C12huVH2+huVL3(P07303) | 1.63E-08 | 1.02E+05 | 1.67E-03 | 0.997 | 0.49 |
| 33C12huVH3+huVL3(P07304) | 4.87E-08 | 7.56E+04 | 3.68E-03 | 0.995 | 0.308 |

**Table 13: Kinetic analysis results of humanized antibodies designed based on maternal mouse antibody 12E12**

| **Sample number** | **KD (M)** | **kd (1/s)** | **ka(1/Ms)** | **R²** | **Rₘₐₓ** |
|---|---|---|---|---|---|
| 12E12-hFc | 3.27E-09 | 2.16E-03 | 6.59E+05 | 9.86E-01 | 7.34E-01 |
| 12E12huVH3+huVL2(P07282) | 6.38E-09 | 4.87E-03 | 7.64E+05 | 9.85E-01 | 5.66E-01 |
| 12E12huVH5+huVL2(P07286) | 1.71E-09 | 1.97E-03 | 1.15E+06 | 9.81E-01 | 3.23E-01 |
| 12E12huVH3+huVL4(P07290) | 5.03E-09 | 4.07E-03 | 8.10E+05 | 9.82E-01 | 5.54E-01 |
| 12E12huVH4+huVL4(P07291) | 4.84E-09 | 3.79E-03 | 7.82E+05 | 9.77E-01 | 6.19E-01 |
| 12E12huVH5+huVL4(P07292) | 3.14E-09 | 3.40E-03 | 1.08E+06 | 9.77E-01 | 4.20E-01 |
| 12E12-huVH6+huVL2(P08937) | 5.28E-09 | 4.49E-03 | 8.49E+05 | 9.78E-01 | 6.02E-01 |
| 12E12-huVH7+huVL2(P08938) | 1.70E-09 | 1.83E-03 | 1.08E+06 | 9.77E-01 | 3.69E-01 |
| 12E12-huVH8+huVL2(P08939) | 3.92E-09 | 4.42E-03 | 1.13E+06 | 9.75E-01 | 4.50E-01 |
| 12E12-huVH6+huVL4(P08940) | 4.03E-09 | 3.40E-03 | 8.44E+05 | 9.75E-01 | 5.98E-01 |
| 12E12-huVH7+huVL4(P08941) | 2.80E-09 | 3.50E-03 | 1.25E+06 | 9.72E-01 | 4.32E-01 |
| 12E12-huVH8+huVL4(P08942) | 3.25E-09 | 4.37E-03 | 1.34E+06 | 9.72E-01 | 4.02E-01 |

The experimental results show that the correlation coefficient R² of all antibodies in the Global fitting mode is greater than 0.95, which meets the system adaptability requirements and the results are reliable.

### 12.3 Flow cytometry study on the binding of humanized antibody to TNFR2 protein on cell membrane

To determine whether the humanized TNFR2 monoclonal antibody of the present disclosure can bind to the TNFR2 protein on the cell membrane, *in vitro* testing was conducted. The human-mouse chimeric antibody was used as a positive control. The experimental method is the same as Example 3.2

### Experimental results and analysis

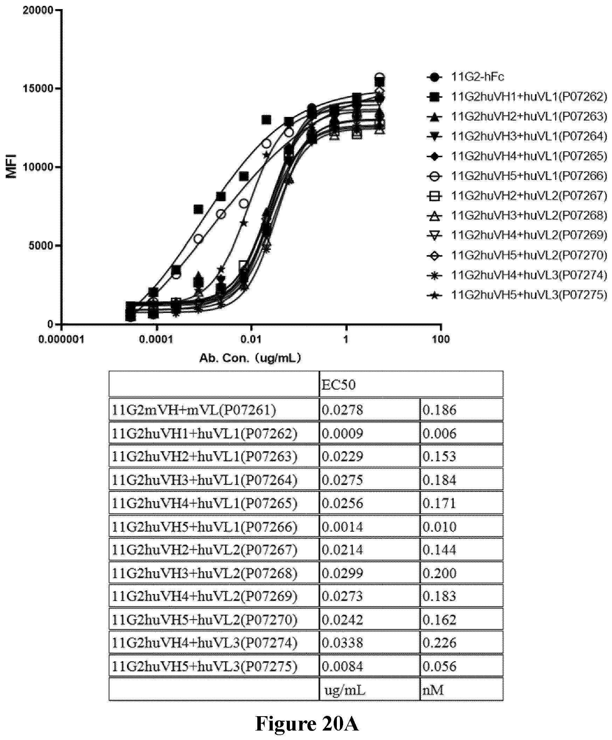

As shown in Figure 20A, 11 strains of 11G2 anti-human TNFR2 humanized monoclonal antibodies (number P07262-P07270, P07274, P07275) bound well to CHO-TNFR2. The human-mouse chimeric 11G2 antibody served as a control, with an EC₅₀ of 0.186 nM. The EC₅₀ of the humanized antibodies binding to CHO-TNFR2 cell membrane was: 0.006 nM, 0.153 nM, 0.184 nM, 0.171 nM, 0.010 nM, 0.144 nM, 0.200 nM, 0.183 nM, 0.162 nM, 0.226 nM, 0.056 nM, respectively.

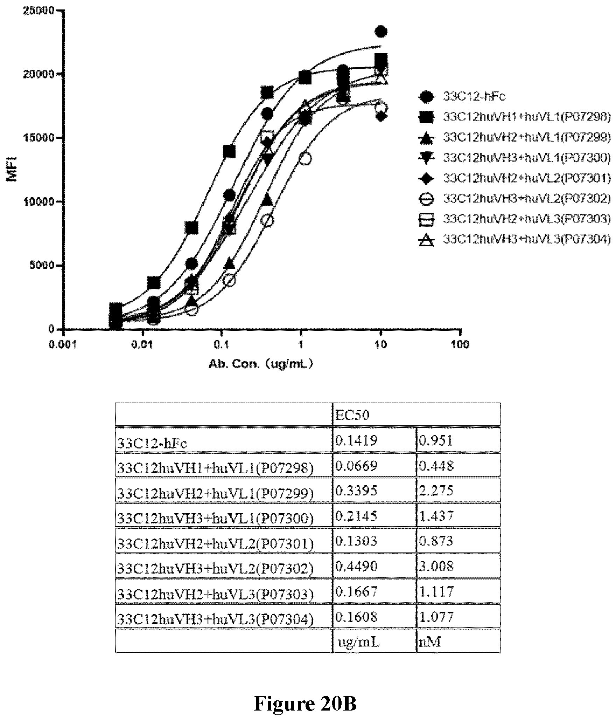

As shown in Figure 20B, 7 strains of 33C12 anti-human TNFR2 humanized monoclonal antibodies (number P07298-P07304) all bound well to CHO-TNFR2. The human-mouse chimeric 33C12 antibody served as a control, with an EC₅₀ of 0.951 nM. The EC₅₀ of humanized 33C12 antibodies binding to CHO-TNFR2 cell membrane was 0.448 nM, 2.275 nM, 1.437 nM, 0.873 nM, 3.008 nM, 1.117 nM, and 1.077 nM, respectively.

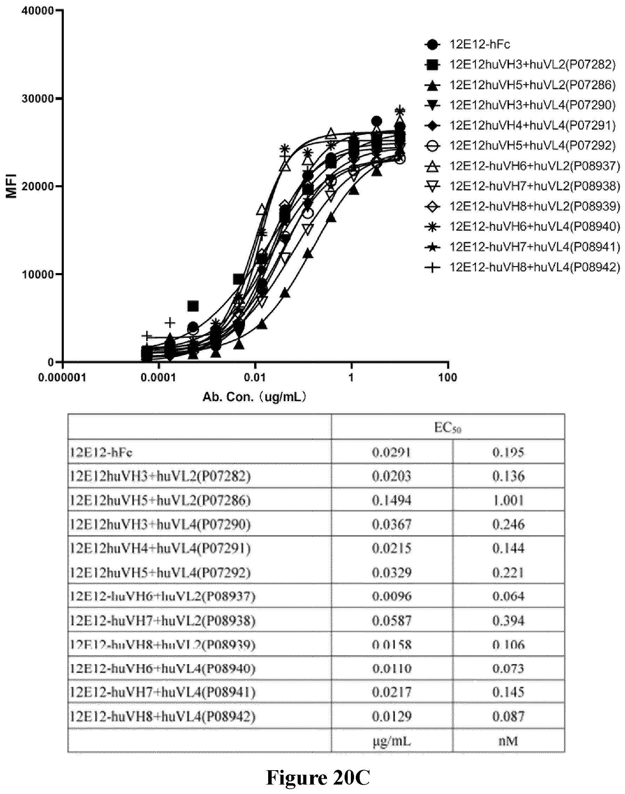

As shown in Figure 20C, 11 strains of 12E12 anti-human TNFR2 humanized monoclonal antibodies all bound well to CHO-TNFR2. The human-mouse chimeric 12E12 antibody served as a control, with an EC₅₀ of 0.195 nM. The EC₅₀ of humanized 12E12 antibodies binding to CHO-TNFR2 cell membranes was 0.136 nM, 1.001 nM, 0.246 nM, 0.144 nM, 0.221 nM, 0.064 nM, 0.394 nM, 0.106 nM, 0.073 nM, 0.145 nM, and 0.087 nM, respectively.

The above results indicate that the recombinant humanized antibody can bind to antigen TNFR2 on the cell membrane, and the binding activity is comparable to that of human-mouse chimeric antibody.

### Example 13. Effect of humanized anti-human TNFR2 monoclonal antibody on T cell activation

The commercially available frozen PBMC cells (Oricellls) were resuscitated and the cell density was adjusted to 5×10⁶ cells/mL. PHA was diluted to 2µg/mL with RPMI1640+10% FBS medium, the antibody to be tested was diluted by the above PHA working solution in gradient to 20 µg/mL, and gently mixed well. The antibody was added to the corresponding well of 96-well plate at 100µL per well, and finally 100 µL of cell suspension solution was added into the corresponding well, gently mixed and incubated for 3 days at 37 °C in an incubator with 5% CO₂. After 3 days, 100µL of the supernatant was absorbed to detect the content of IFNy (Ekosai, catalog number EH008).

For the determination of CD25 expression and the proportion of CD8+/CD4+cells, the cells were collected, washed and resuspended with PBS, then stained with FITC-anti-human CD4 (Biolegend), PE-anti-human CD8a (Biolegend), APC anti huamn-CD25 (Biolegend, 302610). After incubated at 4 °C for 30 minutes, the cells washed and resuspended with PBS for detection.

### Experimental results and analysis

**Table 14. Results of the effects of humanized anti-human TNFR2 monoclonal antibodies based on 11G2 and 33C12 on T cell activation**

| | Antibody conc. (µg/mL) | IFN γ (pg/mL) | Increase% | CD3+CD25+ MFI | CD8+%/ CD4+% |
|---|---|---|---|---|---|
| 11G2huVH1+huVL1 (P07262) | 10 | 12054 | 556% | 18786.7 | 2.33 |
| 11G2huVH2+huVL1 (P07263) | 10 | 6342 | 247% | 14612.8 | 2.23 |
| 11G2huVH3+huVL2 (P07268) | 10 | 7674 | 319% | 15434.4 | 2.32 |
| 33C12huVH1+huVL1 (P07298) | 10 | 5002 | 173% | 16169 | 2.20 |
| 33C12huVH2+huVL3 (P07303) | 10 | 5022 | 174% | 14898.8 | 2.11 |
| 11G2-hFc | 10 | 7754 | 324% | 13939.7 | 2.22 |
| IgG1 | 10 | 1830 | 0% | 11493.2 | 1.82 |

### CD25 expression

One characteristic of T cell activation is the high expression of some CD molecules on the cell surface, such as CD25. The expression of CD25 in 72h cells were analyzed. As shown in the above table, compared with the negative control IgG1, the group treated with the anti-TNFR2 human-mouse chimeric antibody (11G2-hFc) and humanized antibodies showed a significant increase in CD25 expression in cells, indicating that T cells were activated by TNFR2 antibody.

### CD8+/CD4+cell ratio

Meanwhile, the percentage of CD8+ and CD4+ cells in the entire cell population at 72-hour was analyzed and the ratio between the two was calculated. The results showed that after 72 hours, the ratio of CD8+/CD4+ in the anti-TNFR2 antibody treatment group was higher than that in the control group, indicating that the antibody of the present invention mainly promotes the proliferation of CD8+T cells.

### Cytokine IFNγ

After T cell activation, a large amount of cytokines, such as IFNy, are released. The content of IFNy in the cell supernatant at 72-hour was measured. The results showed that compared with the control group, IFNy content of the cell group treated with the antibody of the present invention had a significant increase, with an increase percentage of 173%-556%.

**Table 15. Results of the effect of humanized anti-human TNFR2 monoclonal antibody based on 12E12 on T cell activation**

| | Antibody conc. (µg/mL) | IFN γ (pg/mL) | Increase% |
|---|---|---|---|
| 12E12huVH3+huVL2(P07282) | 10 | 25107.6 | 1086% |
| 12E12huVH4+huVL4(P07291) | 10 | 21498.1 | 915% |
| 12E12huVH6+huVL2(P08937) | 10 | 17317.1 | 718% |
| 12E12huVH6+huVL4(P08940) | 10 | 25693.3 | 1114% |
| 12E12-hFc | 10 | 15883.8 | 650% |
| IgG1 | 10 | 2117.1 | 0% |

The above table shows that the four humanized TNFR2 monoclonal antibody and human-mouse chimeric antibody disclosed herein can promote the production of IFNγ in PHA-activated PBMCs, compared with the control group IgG1, the IFNγ content of the cell group treated with the antibody of the present invention has a significant increase, with an increase percentage of 650%-1114%.

The above experimental results show that the anti-TNFR2 chimeric antibody and the humanized antibody of the present invention can significantly promote the activation of T cells.

All references mentioned in the present invention are incorporated by reference herein, as though individually incorporated by reference herein. In addition, it should be understood that after reading the above teaching content of the present invention, various changes or modifications may be made by those skilled in the art, and these equivalent forms also fall within the scope as defined by the appended claims of the present application.

## Claims

1. A monoclonal antibody or antigen-binding fragment thereof targeting TNFR2, which specifically binds to the cysteine-rich domains (CRDs) of human TNFR2.

2. The monoclonal antibody or antigen-binding fragment thereof according to claim 1, wherein the CRD is the CRD1, CRD2, CRD3, and/or CRD4 of TNFR2.

3. The monoclonal antibody or antigen-binding fragment thereof according to claim 1, wherein
(I) the monoclonal antibody has heavy chain complementary determining regions (VH CDR) 1-3 selected from the following group, light chain complementary determining regions (VL CDR) 1-3 selected from the following group, or a sequence with at least 95% sequence identity with the CDR:
VH CDR1 selected from the group consisting of: SEQ ID NOs: 2, 10, 18, 26, 34, 42, 50, 58, 66;
VH CDR2 selected from the group consisting of: SEQ ID NOs: 3, 11, 19, 27, 35, 43, 51, 59, 67, 87, 88, 90, 95, or 98;
VH CDR3 selected from the group consisting of: SEQ ID NOs: 4, 12, 20, 28, 36, 44, 52, 60, 68;
VL CDR1 selected from the group consisting of: SEQ ID NOs: 6, 14, 22, 30, 38, 46, 54, 62, 70;
VL CDR2 selected from the group consisting of: SEQ ID NOs: 7, 15, 23, 31, 39, 47, 55, 63, 71, 89, 81, 107; and
VL CDR3: selected from the group consisting of: SEQ ID NOs: 8, 16, 24, 32, 40, 48, 56, 64, 72;
and/or
(II) the monoclonal antibody has amino acid sequences of VH CDR1-3 set forth in: SEQ ID NOs: 2, 3, and 4, respectively; SEQ ID NOs: 10, 11 and 12, respectively; SEQ ID NOs: 18, 19 and 20, respectively; SEQ ID NOs: 26, 27, and 28, respectively; SEQ ID NOs: 34, 35, and 36, respectively; SEQ ID NOs: 42, 43, and 44, respectively; SEQ ID NOs: 50, 51, and 52, respectively; SEQ ID NOs: 58, 59, and 60, respectively; SEQ ID NOs: 66, 67, and 68, respectively; SEQ ID NOs: 34, 87, and 36, respectively; SEQ ID NOs: 34, 88, and 36, respectively; SEQ ID NOs: 66, 90, and 68, respectively; SEQ ID NOs: 42, 95, and 44, respectively; SEQ ID NOs: 42, 98, and 44, respectively; and/or
the monoclonal antibody has amino acid sequences of VL CDR1-3set forth in: SEQ ID NOs: 6, 7, and 8, respectively; SEQ ID NOs: 14, 15 and 16, respectively; SEQ ID NOs: 22, 23, and 24, respectively; SEQ ID NOs: 30, 31, and 32, respectively; SEQ ID NOs: 38, 39, and 40, respectively; SEQ ID NOs: 46, 47, and 48, respectively; SEQ ID NOs: 54, 55, and 56, respectively; SEQ ID NOs: 62, 63, and 64, respectively; SEQ ID NOs: 70, 71, and 72, respectively; SEQ ID NOs: 38, 89, and 40, respectively; SEQ ID NOs: 70, 91, and 72, respectively; SEQ ID NOs: 46, 107, and 48, respectively; and/or
(III) the monoclonal antibody has heavy chain complementary determining regions (VH CDR) 1-3 and light chain complementary determining regions (VL CDR) 1-3 selected from the group consisting of:
(a) the amino acid sequences of VH CDR1-3 are set forth in SEQ ID NOs: 2-4, respectively, and the amino acid sequences of VL CDR1-3 are set forth in SEQ ID NOs: 6-8, respectively;
(b) the amino acid sequences of VH CDR1-3 are set forth in SEQ ID NOs: 10-12, respectively, and the amino acid sequences of VL CDR1-3 are set forth in SEQ ID NOs: 14-16, respectively;
(c) the amino acid sequences of VH CDR1-3 are set forth in SEQ ID NOs: 18-20, respectively, and the amino acid sequences of VL CDR1-3 are set forth in SEQ ID NOs: 22-24, respectively;
(d) the amino acid sequences of VH CDR1-3 are set forth in SEQ ID NOs: 26-28, respectively, and the amino acid sequences of VL CDR1-3 are set forth in SEQ ID NOs: 30-32, respectively;
(e) the amino acid sequences of VH CDR1-3 are set forth in SEQ ID NOs: 34-36, respectively, and the amino acid sequences of VL CDR1-3 are set forth in SEQ ID NOs: 38-40, respectively;
(f) the amino acid sequences of VH CDR1-3 are set forth in SEQ ID NOs: 42-44, respectively, and the amino acid sequences of VL CDR1-3 are set forth in SEQ ID NOs: 46-48, respectively;
(g) the amino acid sequences of VH CDR1-3 are set forth in SEQ ID NOs: 50-52, respectively, and the amino acid sequences of VL CDR1-3 are set forth in SEQ ID NOs: 54-56, respectively;
(h) the amino acid sequences of VH CDR1-3 are set forth in SEQ ID NOs: 58-60, respectively, and the amino acid sequences of VL CDR1-3 are set forth in SEQ ID NOs: 62-64, respectively;
(i) the amino acid sequences of VH CDR1-3 are set forth in SEQ ID NOs: 66-68, respectively, and the amino acid sequences of VL CDR1-3 are set forth in SEQ ID NOs: 70-72, respectively;
(j) the amino acid sequences of VH CDR1-3 are set forth in SEQ ID NOs: 34, 87 and 36, respectively, and the amino acid sequences of VL CDR1-3 are set forth in SEQ ID NOs: 38-40, respectively;
(k) the amino acid sequences of VH CDR1-3 are set forth in SEQ ID NOs: 34, 87 and 36, respectively, and the amino acid sequences of VL CDR1-3 are set forth in SEQ ID NOs: 38, 89 and 40, respectively;
(l) the amino acid sequences of VH CDR1-3 are set forth in SEQ ID NOs: 34, 88 and 36, respectively, and the amino acid sequences of VL CDR1-3 are set forth in SEQ ID NOs: 38-40, respectively;
(m) the amino acid sequences of VH CDR1-3 are set forth in SEQ ID NOs: 34, 88 and 36, respectively, and the amino acid sequences of VL CDR1-3 are set forth in SEQ ID NOs: 38, 89 and 40, respectively;
(o) the amino acid sequences of VH CDR1-3 are set forth in SEQ ID NOs: 66, 90 and 68, respectively, and the amino acid sequences of VL CDR1-3 are set forth in SEQ ID NOs: 70-72, respectively;
(p) the amino acid sequences of VH CDR1-3 are set forth in SEQ ID NOs: 66, 90 and 68, respectively, and the amino acid sequences of VL CDR1-3 are set forth in SEQ ID NOs: 70, 91 and 72, respectively;
(q) the amino acid sequences of VH CDR1-3 are set forth in SEQ ID NOs: 42, 95 and 44, respectively, and the amino acid sequences of VL CDR1-3 are set forth in SEQ ID NOs: 46, 47 and 48, respectively;
(r) the amino acid sequences of VH CDR1-3 are set forth in SEQ ID NOs: 42, 98 and 44, respectively, and the amino acid sequences of VL CDR1-3 are set forth in SEQ ID NOs: 46, 47, and 48, respectively.

4. The monoclonal antibody or antigen-binding fragment thereof according to claim 1, wherein
the monoclonal antibody has a heavy chain variable region (VH) selected from the group consisting of
the VH amino acid sequence set forth in any one of SEQ ID NOs: 1, 9, 17, 25, 33, 41, 49, 57, 65, or a sequence with at least 70% sequence identity (such as SEQ ID NO: 73, 74, 75, 76, or 77; SEQ ID NO: 81, 82, or 83; SEQ ID NO: 92, 93, 94, 96, 97, 99, 100, or 101); and/or
the monoclonal antibody has a light chain variable region (VL) selected from the group consisting of
the VL amino acid sequence set forth in any one of SEQ ID NOs: 5, 13, 21, 29, 37, 45, 53, 61, and 69, or a sequence with at least 70% sequence identity (such as SEQ ID NO: 78, 79, or 80; SEQ ID NO: 84, 85, and 86; SEQ ID NO: 102, 103, 104, 105, or 106);
for example, the monoclonal antibody has the VH and VL sequences selected from the group consisting of:
(A) the VH amino acid sequence is SEQ ID NO: 1 or a sequence with at least 70% sequence identity, and the VL amino acid sequence is SEQ ID NO: 5 or a sequence with at least 70% sequence identity;
(B) the VH amino acid sequence is SEQ ID NO: 9 or a sequence with at least 70% sequence identity, and the VL amino acid sequence is SEQ ID NO: 13 or a sequence with at least 70% sequence identity;
(C) the VH amino acid sequence is SEQ ID NO: 17 or a sequence with at least 70% sequence identity, and the VL amino acid sequence is SEQ ID NO: 21 or a sequence with at least 70% sequence identity;
(D) the VH amino acid sequence is SEQ ID NO: 25 or a sequence with at least 70% sequence identity, and the VL amino acid sequence is SEQ ID NO: 29 or a sequence with at least 70% sequence identity;
(E) the VH amino acid sequence is SEQ ID NO: 33 or a sequence with at least 70% sequence identity (such as SEQ ID NO: 73, 74, 75, 76 or 77), and the VL amino acid sequence is SEQ ID NO: 37 or a sequence with at least 70% sequence identity (such as SEQ ID NO: 78, 79, or 80);
(F) the VH amino acid sequence is SEQ ID NO: 41 or a sequence with at least 70% sequence identity (such as SEQ ID NO: 92, 93, 94, 96, 97, 99, 100, or 101), and the VL amino acid sequence is SEQ ID NO: 45 or a sequence with at least 70% sequence identity (such as SEQ ID NO: 102, 103, 104, 105, or 106);
(G) the VH amino acid sequence is SEQ ID NO: 49 or a sequence with at least 70% sequence identity, and the VL amino acid sequence is SEQ ID NO: 53 or a sequence with at least 70% sequence identity;
(H) the VH amino acid sequence is SEQ ID NO: 57 or a sequence with at least 70% sequence identity, and the VL amino acid sequence is SEQ ID NO: 61 or a sequence with at least 70% sequence identity; or
(I) the VH amino acid sequence is SEQ ID NO: 65 or a sequence with at least 70% sequence identity (such as SEQ ID NO: 81, 82, or 83), and the VL amino acid sequence is SEQ ID NO: 69 or a sequence with at least 70% sequence identity (such as SEQ ID NO: 84, 85, or 86).

5. The monoclonal antibody or antigen-binding fragment thereof according to claim 1, wherein the monoclonal antibody is a humanized antibody, such as selected from a chimeric antibody, a CDR transplant antibody, a homologous substitution antibody, a surface remodeling antibody, a compensatory mutation antibody, and site-specific conservative humanized antibody; and/or
the monoclonal antibody or antigen-binding fragment thereof comprises: antibody, Fab, Fab', F(ab')₂, Fd, single-chain Fv or scFv, disulfide-linked Fv, V-NAR domain, IgNar, intrabody, IgGΔCH₂, mini-antibody, F(ab')₃, tetra-antibody, tri-antibody, bispecific antibody, single-domain antibody, DVD-Ig, Fcab, mAb₂, (scFv)₂ or scFv-Fc.

6. The monoclonal antibody or antigen-binding fragment thereof according to claim 5, wherein the monoclonal antibody is a chimeric antibody, wherein:
the constant region (C region) is a human constant region, such as the heavy chain constant region is a human IgG (such as IgG1, IgG2, IgG3 or IgG4) and/or the light chain constant region is a human κ or λ;
the variable region (V region) is a mouse variable region, for example, the chimeric antibody further has the CDR according to claim 2 and/or the VH and VL according to claim 3.

7. The monoclonal antibody or antigen-binding fragment thereof according to claim 1, wherein the monoclonal antibody is a CDR transplant antibody comprising the sequences of VH CDR 1-3 and VL CDR 1-3 according to claim 3.

8. The monoclonal antibody or antigen-binding fragment thereof according to claim 1, wherein the monoclonal antibody is a surface remodeling antibody, wherein the monoclonal antibody is obtained by humanization and substitution of one or more amino acid residues in VH and VL according to claim 4, wherein the amino acid substitution is located or not located in CDR.

9. The monoclonal antibody or antigen-binding fragment thereof according to any one of claims 1-8, which has one or more features selected from the following group:
(i) specifically binds to human TNFR2 *in vivo* or *in vitro,* for example, the EC₅₀ of the binding affinity with human TNFR2 detected by ELISA is 0.001nM-50nM, 0.002-20nM, or 0.002-10nM;
(ii) binds to human and/or non-human primate mammalian TNFR2, but not to mouse TNFR2.

10. The monoclonal antibody or antigen-binding fragment thereof according to any one of claims 1-8, which is an immunoactive antibody or fragment;
for example, the monoclonal antibody or antigen-binding fragment thereof has one or more functions selected from the group consisting of: inhibiting the proliferation and/or activity of regulatory T cells (Treg), promoting the proliferation and/or activity of effector T cells (Teff), inhibiting tumor cells, promoting inflammation, and inhibiting infection; and/or
for example, the monoclonal antibody or antigen-binding fragment thereof has one or more functions selected from the group consisting of enhancing immune response; anti-tumor, preferably TNFR2 expression or overexpression types, such as lung cancer, adrenal cortex cancer, bladder urothelial carcinoma, cervical cancer, cholangiocarcinoma, colon cancer, esophageal cancer, glioma, head and neck squamous cell carcinoma, renal clear cell carcinoma, renal papillary cell carcinoma, acute myeloid leukemia, low-grade brain glioma, hepatocellular carcinoma, ovarian cancer, pancreatic adenocarcinoma, pheochromocytoma and paraganglioma, prostate cancer, rectal adenocarcinoma, sarcoma, melanoma, gastric adenocarcinoma, testicular cancer, thyroid cancer, uterine cancer; anti-infection, such as inducing inflammatory reactions.

11. The monoclonal antibody or antigen-binding fragment thereof according to any one of claims 1-8, which is an immunosuppressive antibody or fragment;
for example, the antibody or antigen-binding fragment thereof has one or more functions selected from the group consisting of: promoting the proliferation and/or activity of regulatory T cells (Treg), inhibiting the proliferation and/or activity of effector T cells (Teff), downregulating immune activity, inhibiting immune response, and suppressing inflammatory response; and/or
for example, the antibody or antigen binding fragment thereof has one or more functions selected from the group consisting of: inhibiting immune response, anti-autoimmune disease, anti-cytokine storm, anti-allergic disease, anti-graft versus host.

12. A nucleic acid molecule encoding any one of the monoclonal antibody or antigen-binding fragment thereof according to any one of claims 1-11, or a vector or a host cell containing the nucleic acid molecule,

13. An immunoconjugate comprising:
(a) the monoclonal antibody or antigen-binding fragment thereof according to any one of claims 1-11;
(b) a coupling moeity selected from the group consisting of: a drug, a toxin, a cytokine, a radioactive isotope or an enzyme; and
(c) optionally, a linker.

14. A chimeric antigen receptor comprising an extracellular domain and an intracellular domain, wherein the extracellular domain comprises the monoclonal antibody or antigen-binding fragment thereof according to any one of claims 1-11.

15. The chimeric antigen receptor according to claim 14, wherein:
the antibody or antigen-binding fragment thereof comprises or is composed of scFv or VH sdAb;
the intracellular domain encoding one or more selected from the group consisting of: ITAM domain, CD3ζ, CD28, 4-1BB, OX40, CD27, ICOS, or a combination thereof, such as (CD28+CD3ζ), (CD28+CD27+CD3ζ), (CD28+OX40+CD3ζ), (CD28+4-1BB+CD3ζ), (CD28+CD27+OX40+CD3ζ), (CD28+4-1BB+CD27+CD3ζ), (CD28+4-1BB+OX40+CD3ζ), (4-1BB+CD3ζ), (4-1BB+OX40+CD3ζ), (4-1BB+CD27+CD3ζ), (CD27+CD3ζ), (CD27+OX40+CD3ζ), (CD28Δ+CD3ζ), (CD28Δ+CD27+CD3ζ), (CD28Δ+ OX40+CD3ζ), (CD28Δ+4-1BB+CD3ζ), (CD28Δ+4-1BB+OX40+CD3ζ), (CD28Δ +CD27+OX40+CD3ζ), (CD28Δ+4-1BB+CD27+CD3ζ), (4-1BB+ICOS+CD3ζ), (CD28+ICOS+CD3ζ), (ICOS+CD3ζ); and/or
the extracellular domain of the chimeric antigen receptor further comprises a hinge region, such as the hinge region derived from the hinge of IgG or CD8α/CD28 extracellular domain, such as selected from: IgG4 Fc Δ EQ, IgG4 Fc Δ Q, (t-12AA+t-20AA), mKate, phiLov, dsRed, Venus, eGFP, CH3 HA, (CD8α+t-20AA), dual t-20 AA, (t-20AA+CD8α), (CD8α + Leucine zipper Basepl), (CD8α + Leucine zipper Acid1), 2D3, CD8a or IgG4 Fc; and/or
the chimeric antigen receptor further comprises a transmembrane domain connecting the extracellular domain and the intracellular domain, such as derived from the α, β or ζ chain of T-cell receptor, such as the transmembrane region of CD28, CD3ε, CD45, CD4, CD5, CDS, CD9, CD16, CD22, CD33, CD37, CD64, CD80, CD86, CD134, CD137, and CD154.

16. A nucleic acid molecule and a construct or a vector containing the nucleotide molecule, wherein the nucleic acid molecule comprises a coding sequence of the chimeric antigen receptor according to claims 14 or 15.

17. A transformed immune cell, which expresses the chimeric antigen receptor according to claims 14 or 15, or is transformed by the nucleic acid molecule, construct, or vector according to claim 16, for example, the immune cell is selected from T cells, such as αβ T cell, γδ T cell or NK T cell or T cell derived from pluripotent cell.

18. A composition comprising:
the monoclonal antibody or antigen-binding fragment thereof according to any one of claims 1-11, the nucleic acid molecule, vector or host cell according to claim 12, or the immuneconjugate according to claim 13, the chimeric antigen receptor according to claims 14-15, the nucleic acid molecule, construct or vector according to claim 16, and/or the transformed immune cell according to claim 17; and
optionally, a physiologically or pharmaceutically acceptable carrier or excipient;
for example, the composition is a pharmaceutical composition, which further comprises a pharmaceutically acceptable carrier or excipient; a detection kit, which further comprises the reagent required to detect TNFR2 level or the activity or related pathway thereof.

19. Use of the monoclonal antibody or antigen-binding fragment thereof according to any one of claims 1-11, the nucleic acid molecule, vector or host cell according to claim 12, the immunoconjugate according to claim 13, the chimeric antigen receptor according to claims 14-15, the nucleic acid molecule, construct or vector according to claim 16, the transformed immune cell according to claim 17 and/or the composition according to claim 18 in the preparation of a product that positively regulates the activity of immune cell and/or improves immune response, such as the use in the preparation of a drug for preventing and/or treating tumors, infections or infectious diseases.

20. Use of the monoclonal antibody or antigen-binding fragment thereof according to any one of claims 1-11, the nucleic acid molecule, vector or host cell according to claim 12, the immuneconjugate according to claim 13, the chimeric antigen receptor according to claims 14-15, the nucleic acid molecule, construct or vector according to claim 16, the transformed immune cell according to claim 17 and/or the composition according to claim 18 in the preparation of a product that negatively regulates the activity of immune cell and/or reduces immune response,
such as the use in the preparation of a drug for the prevention and/or treatment of autoimmune diseases, cytokine storms, or allergic diseases.

21. Use of the monoclonal antibody or antigenbinding fragment according to any one of claims 1-11, the nucleic acid molecule, vector or host cell according to claim 12, the immunoconjugate according to claim 13, or the composition according to claim 18 in the preparation of a reagent kit for detecting the presence, level or activity of TNFR2.
